# EUROPEAN PATENT APPLICATION

(11) **EP 1 944 314 A1**
(43) Date of publication of application: **16.07.2008**
(21) Application number: 07000507.9
(22) Date of filing: 11.01.2007
(51) Int. Cl.: C07K 14/47, G01N 33/68, G01N 33/50

(54) **Diagnosis of Alzheimer's disease and other neurodementing disorders**

(71) Applicant: Philipps-Universität Marburg, 35037 Marburg (DE); Universität Konstanz, 78464 Konstanz (DE)
(72) Inventor: Dodel, Richard, 35096 Weimar a.d. Lahn (DE); Stefanescu, Raluca, 78467 Konstanz (DE); Bacher, Michael, 35091 Cölbe (DE); Manea, Marilena, 78467 Konstanz (DE); Przybylski, Michael, 65468 Trebur1 (DE)
(74) Representative: Dehmel, Albrecht

(57) **Abstract**

The present invention relates to a polypeptide comprising an amino acid sequence of an Aβ peptide, wherein the Aβ peptide has at least the sequence according to SEQ ID NO: 2 and at most the sequence according to SEQ ID NO: 4. Furthermore the present invention relates to methods of the diagnosis of Alzheimer's disease and other neurodementing diseases.

SEQUENCE LISTING

<110>  Universitaet Konstanz
       Rheinische Friedrich-Wilhelms-Universitaet Bonn

<120>  Diagnosis of Alzheimer's Disease and other neurodementing
       disorders

<130>  UBK-001 EP

<160>  5

<170>  PatentIn version 3.3

<210>  1
<211>  40
<212>  PRT
<213>  Homo sapiens

<400>  1

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val
        35                  40

<210>  2
<211>  17
<212>  PRT
<213>  Homo sapiens

<400>  2

Ala Glu Asp Val Gly Ser Asn Lys Gly Ala Ile Ile Gly Leu Met Val
1               5                   10                  15

Gly

<210>  3
<211>  7
<212>  PRT
<213>  Homo sapiens

<400>  3

Phe Arg His Asp Ser Gly Tyr
1               5

<210>  4
<211>  29
<212>  PRT
<213>  Homo sapiens

<400>  4

Val His His Gln Lys Leu Val Phe Phe Ala Glu Asp Val Gly Ser Asn
1               5                   10                  15

Lys Gly Ala Ile Ile Gly Leu Met Val Gly Gly Val Val
            20                  25

<210>  5
<211>  20
<212>  PRT
<213>  Homo sapiens

<400>  5

Asp Ala Glu Phe Arg His Asp Ser Gly Tyr Glu Val His His Gln Lys
1               5                   10                  15

Leu Val Phe Phe
            20

## Description

### Field of the Invention

The present invention relates to a polypeptide comprising an amino acid sequence of an Aβ peptide, wherein the Aβ peptide has at least the sequence according to SEQ ID NO: 2 and at most the sequence according to SEQ ID NO: 4. Furthermore the present invention relates to methods of diagnosis of Alzheimer's disease and other neurodementing disorders.

### Description of Related Art

Alzheimer's disease (AD), the most common form of dementia among elderly population (prevalence: 1000/100,000 >65 y), represents the fourth leading cause of death in the developed world. Cortical atrophy, neuronal loss, region-specific amyloid deposition, neuritic plaques, and neurofibrillary tangles are key neuropathological features in AD brain. These alterations are thought to be linked to cognitive decline which clinically defines AD. Within these markers, neuritic plaques are amyloid immunoreactive, thioflavin positive, and accompanied by astrogliosis, microgliosis, cytoskeletal changes, and synaptic loss. The degree of neuritic degeneration within plaques correlates with clinical parameters of dementia. Neuritic plaques are spherical, multicellular lesions that are usually found in moderate to large numbers in limbic structures and associated neocortices of the AD brain. These plaques are comprised of extracellular deposits of amyloid-β peptide(s) (Aβ) that include abundant amyloid fibrils intermixed with non-fibrillary forms of the peptide. Such plaques also contain variable numbers of activated microglia that are often situated very near the fibrillar amyloid core, as well as reactive astrocytes surrounding the core.

The major constituent of the neuritic plaque, β-amyloid polypeptide (Aβ), arises from a larger precursor protein, the amyloid precursor protein (APP) (Kang, et al., 1987; Tanzi, et al., 1987). Aβ is produced by normal cells and can be detected as a circulating peptide in the plasma and cerebrospinal fluid (CSF) of healthy humans. Although the physiological role of the amyloid precursor protein (APP) in the brain is not well understood, missense mutations in APP confer autosomal dominant inheritance of AD (FAD), and shed light on potentially important pathogenic mechanism(s). The accumulation of Aβ, a 39-42 amino acid proteolytic product of APP, in neuritic plaques, which at autopsy fulfill the neuropathological criteria for a definitive diagnosis of AD, is thought to be causative for disease progression. A major Aβ cleavage product of APP is the Aβ(1-42) polypeptide, but Aβ peptides shorter at the C-terminus (39 to 41) are also produced by the proteolytic (γ-secretase) cleavage in the membrane. The N-terminal part of Aβ(1-42) is localized in the extracellular region of APP, and the major C-terminal part of the Aβ peptide is contained within the transmembrane domain.

Missense mutations, in APP associated with FAD, occur in proximity to the Aβ domain and result in an increase in the production of the 4kDa Aβ peptide. In AD, it has been postulated that increased synthesis and/or a decreased clearance of Aβ may lead to amyloid plaque deposition and subsequently to the neuropathological changes associated with the disease. In vitro studies, using synthetic Aβ peptide(s), have shown that neurotoxicity is dependent on Aβ being fibrillar and predominantly in a β-pleated sheet conformation.

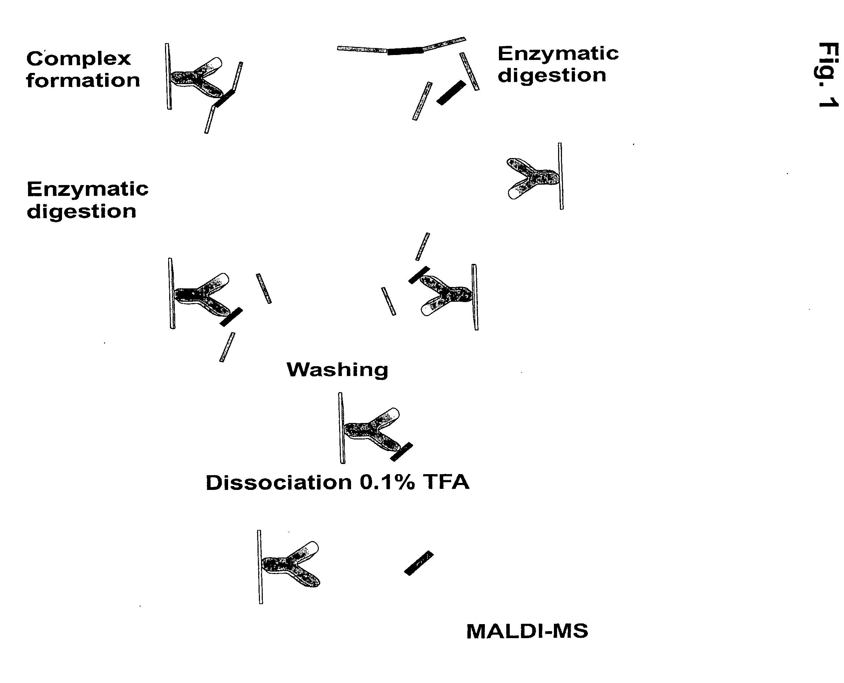

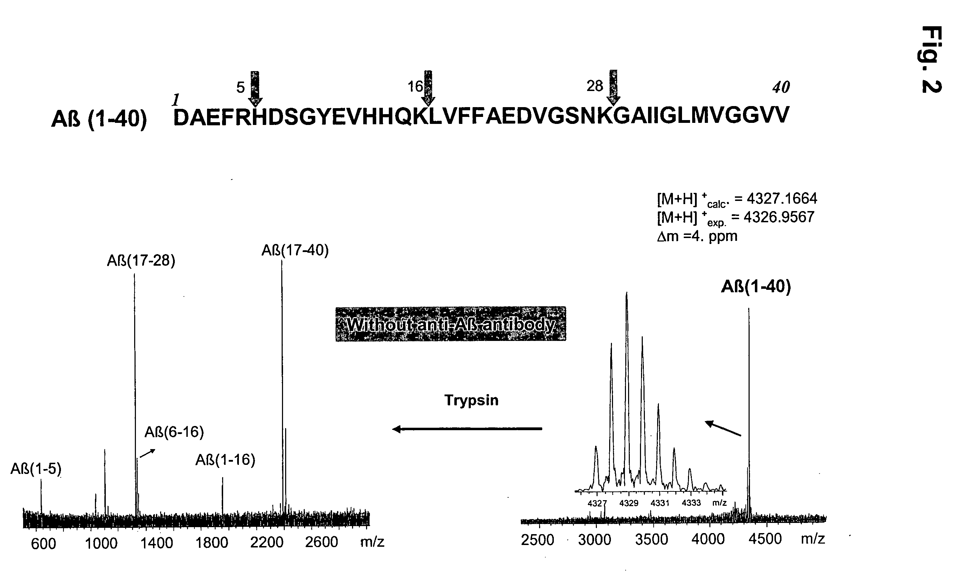

The accumulation of extracellular plaques containing the neurotoxic amyloid peptide fragment (Aβ) of β-amyloid precursor protein (APP), as the major product, is one of the characteristics of Alzheimer's disease (AD). Although APP has been recognised as a key molecule for AD, the molecular (patho-) physiological degradation and proteolytic pathways of APP, and cellular interactions and biochemical fate of Aβ peptide(s) are still unclear. Despite the lack of details on degradation pathways and cellular transport for the formation and deposition of Aβ-derived plaques, recent studies elucidated in more detail the properties of autoantibodies which are directed against the Aβ peptide (Du et al., 2001; Dodel et al., 2002). Recently, antibodies directed against Aβ have been produced upon immunisation with Aβ(1-42) and their recognition properties were investigated in more detail. This work resulted in the identification of a specific Aβ-epitope recognised by the antibodies generated in transgenic AD mice (McLaurin et al., 2002; Przybylski et al., 2003). These results have been obtained by using selective proteolytic excision technologies (Epitope-Excision) in combination with high resolution mass spectrometry (FTICR-MS) as bioanalytical tools of high sensitivity and specificity for the identification of antigen epitopes (Macht et al 1996; Suckau et al 1992; Macht et al. 2004; see Figures 1, 2). Using mass spectrometric epitope excision of the immobilised Aβ-antigen-immune complex, the epitope was identified to consist of the residues (4-10) (FRHDSGY) of Aβ(1-42). The selectivity of this recognition structure was ascertained by elucidation of the identical epitope from AD plaques, Aβ42 extracts from Aβ-protofibrils, chemically synthesised Aβ42, and other (Aβ-independent) polypeptides comprising the N-terminal Aβ sequence (Przybylski et al. 2003).

The major current diagnostics of AD can be grouped into (i), determinations for genetic risk factors or mutations (mainly for FAD cases, but not for sporadic AD diagnostics); (ii), neuroimaging methods; and (iii), diagnostics based on biochemical/biological markers. Present work on the development of diagnostics procedures based on biomarkers have been mainly focused on, and limited to CSF, which has the principal disadvantage that such methods require elaborate, invasive material such as CSF. A major problem associated with brain-derived biomarkers is that clinically examined controls often also include subjects with preclinical AD pathology. Further, current available biomarkers have the major disadvantage of low specificity. Similar disadvantages have been noted for a series of proteins expressed preliminary in the frontal cortex, identified by brain proteomics approaches, as potential brain biomarkers arising from presumed alterations of blood brain barrier in AD.

Studies on biomarkers in plasma and serum have been performed mainly with determinations of SP and NFT components, e.g. the Aβ peptides Aβ(1-40) (SEQ ID NO: 1) and Aβ(1-42) (found with elevated levels) and hyperphosphorylated Tau-protein. However the specificity of Aβ determinations, and application for early and differential diagnostics has been considered uncertain, the same is the case for protein Tau determinations which has been described as a marker of already progressing neurodegeneration.

Thus, the inventors faced the problem to provide a highly specific method for diagnosis and progression of Alzheimer's disease while being still convenient to perform.

The inventors have solved the problem by way of the subject matter set forth in the claims.

### Brief Description of the figures

The following figures form part of the present specification and are included to further demonstrate certain aspects of the present invention. The invention may be better understood by reference to one or more of these figures in combination with the detailed description of specific embodiments presented herein.
Figure 1: Principle of epitope-excision and epitope extraction for mass spectrometric epitope identification. Antibody immunoglobulin with native, disulfide-bonded is generally highly resistant to proteolytic digestion by endoproteases (e.g., trypsin, chymotrypsin, AspN-protease), and the epitope region of antigen polypeptides comprising the epitope-paratope interaction structure is generally protected from proteolytic degradation in the immune complex, while the free nonbinding regions are amenable to digestion. Thus, the epitope sequence remaining bound to the antibody after proteolytic removal and washing away nonbinding structures is then dissociated from the antibody and identified by mass spectrometry. Both electrospray-ionisation (ESI) and matrix-assisted laser desorption-ionisation (MALDI) have been found useful mass spectrometric methods, and have been applied successfully for epitope identifications. "TFA" means trifluoroacetic acid. "MALDI-MS" stands for matrix-assisted laser desorption-ionisation mass spectrometry.
Figure 2: Mass spectrometric identification of proteolytic peptide fragments of free soluble Aβ peptide. Without the complexation by antibody binding, digestion of Aβ(1-40) by trypsin leads to formation of all peptide fragments expected according to the proteolytic cleavage specificity (Aβ(1-5), Aβ(6-16), Aβ(17-28), Aβ(29-40)). Mass spectrometric analysis is performed by high resolution MALDI-Fourier-transform-ion cyclotron resonance (MALDI-FTICR-MS), which provides spectra at approximately 100,000 mass resolution with complete isotope resolution of ions and mass determination accuracies of typically 1-5 ppm. All FTICR-MS spectra were obtained with a Bruker (Bruker Daltonik, Bremen, Germany) Apex II 7T FT-ICR mass spectrometer equipped with an Apollo II electrospray/nanoelectrospray multiport ion source and an external Scout 100 fully-automated X-Y target stage MALDI source with pulsed collision gas. The pulsed nitrogen laser is operated at 337 nm. Ions generated by laser shots were accumulated in the hexapole for 0.5-1 sec at 15 V and extracted at -7 V into the analyzer cell. A 100 mg/ml solution of 2,5-dihydroxybenzoic acid (DHB, Aldrich, Germany) in acetonitrile: 0.1% TFA in water (2:1) was used as the matrix. 0.5 µl of sample solution were mixed on the stainless-steel MALDI sample target and allowed to dry. Typical ESI conditions were ~2 kV needle voltage and 100 nA spray current. Ions were accumulated in a hexapole for 2 sec and then transferred into the cylindrical ICR cell. "ppm" stands for parts per million. "m/z" indicates the mass-to-charge-ratio.

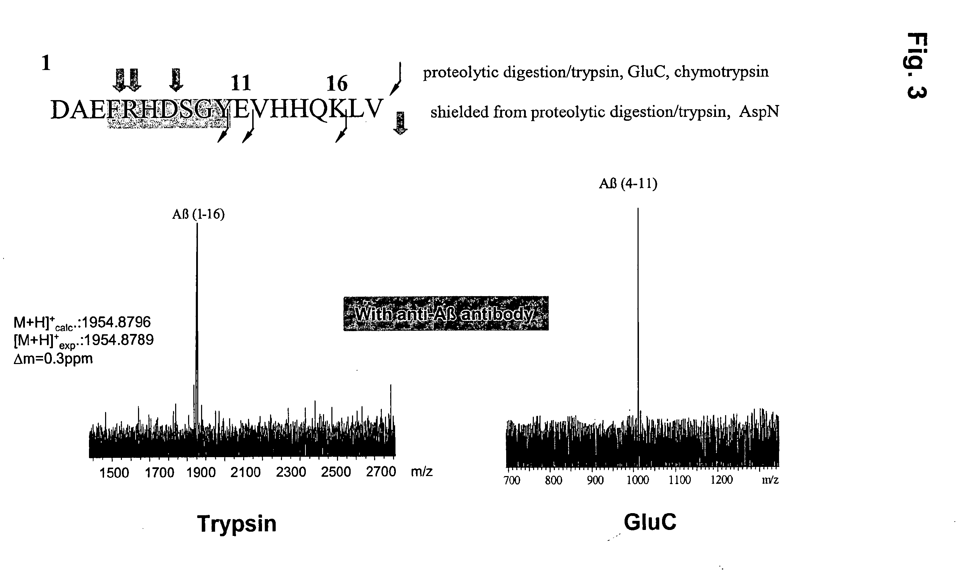

Figure 3: Epitope Identification of Amyloid Plaque Specific Antibody by MALDI-FTICR-MS: Mass spectrometric identification of N-terminal Aβ-epitope recognised by plaque-specific antibody produced upon active immunisation of transgenic mice with Aβ(1-42) or Aβ(1-42)-derived aggregates. The immobilised, purified antibody was incubated with Aβ(1-40), Aβ(1-42), and the immune complex subjected to epitope excision by proteases trypsin, chymotrypsin, Glu-C protease and AspN-protease. The left spectrum shows the fragment, Aβ(1-16) remaining bound after trypsin digestion, the right spectrum shows Aβ(1-11) after epitope excision using GluC-protease. Black small arrows in the Aβ sequence shown denote cleavages identified by epitope excision, fat grey arrows denote substrate cleavage sites on Aβ that were found shielded upon antibody binding. Identical Aβ(4-10) epitope sequences were identified with soluble Aβ-plaques and -protofibrils bound as antigens, and from a mouse anti-Aβ(1-16) peptide monoclonal antibody (Bachem-Peninsula Laboratories, San Francisco).

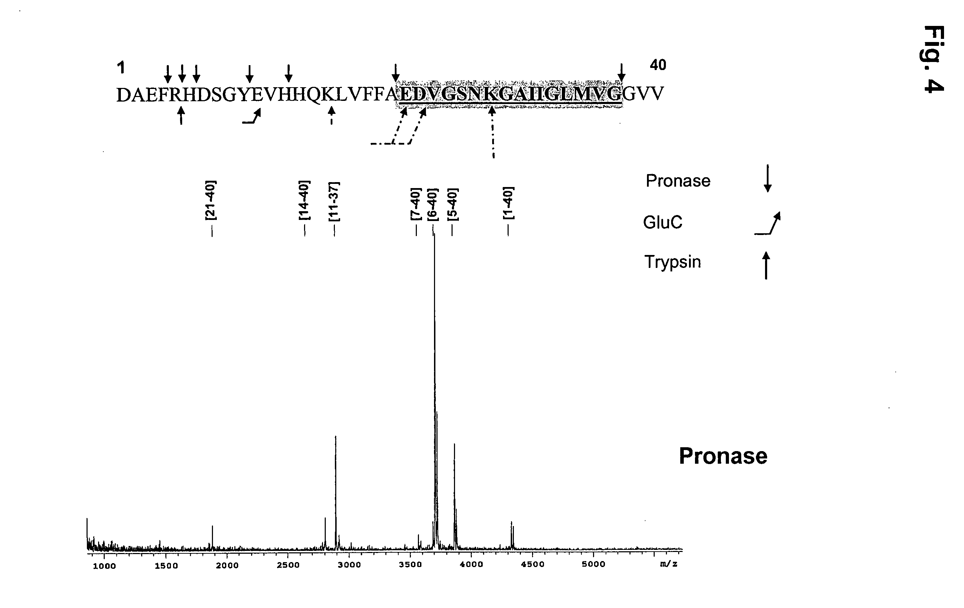

Figure 4: Identification of Aβ (21-37) as the epitope recognised by human Aβ-autoantibodies by epitope excision-mass spectrometry. The upper graph shows the sequence of Aβ(1-40), with cleavages by different proteases indicated by black arrows. Peptide fragments denoted by solid black arrows above the Aβ-sequence were identified after epitope excision using pronase; peptide fragments denoted by black dotted arrows underneath the Aβ sequence were found by epitope excision using trypsin and GluC-protease (R5, E11, K16); note that Arg-5 is completely shielded in the immune complex with the plaque-specific antibody, while completely amenable to cleavage in the immune complex with the Aβ-autoantibody. Cleavage positions, observed in free Aβ, indicated by broken arrows were found shielded after binding of Aβ-autoantibody (GluC: E22, D23; trypsin: K28). The MALDI-MS analysis upon partial digestion (2 hrs) with pronase is shown here for illustration.

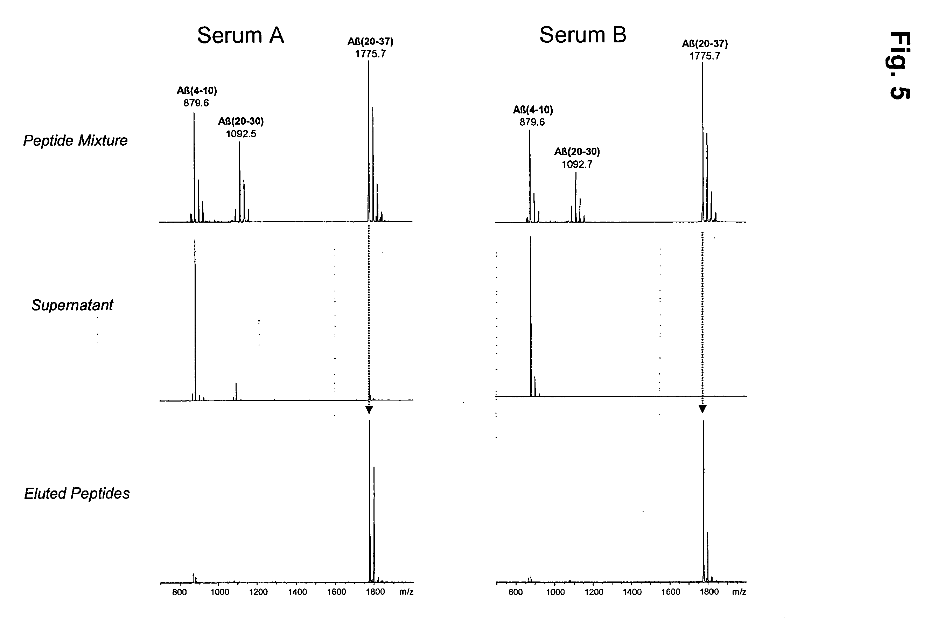

Figure 5: Molecular confirmation of epitope recognition specificity of Aβ-autoantibody. Illustrated is the affinity of 3 synthetic Aβ-polypeptides Aβ(4-10), Aβ(20-30) and Aβ(20-37) towards anti-Aβ-autoantibodies isolated from serum of healthy (non-AD control individuals) donors, A and B, by MALDI-mass spectrometry. Affinity-purified antibodies were immobilised on NHS-sepharose as described in Example 1. Equimolar mixtures (5 µmol mixtures of synthetic Aβ-peptides in aqueous PBS buffer solution, pH 7, were bound to the antibodies after mass spectrometric analysis (s. MALDI-MS of peptide mixture, upper panel). MALDI-MS of the supernatant washing fraction revealed the N-terminal Aβ(4-10) epitope signal as the predominant ion (confirming the lack of binding of N-terminal Aβ; middle panel), and washing was continued until no MS signal was detectable. After elution with 0.1 % trifluoroacetic acid, the Aβ(20-37) peptide was identified as the only polypeptide capable of binding to the autoantibodies (lower panel). All MS determinations were made with a Bruker Bilflex MALDI-TOF spectrometer.

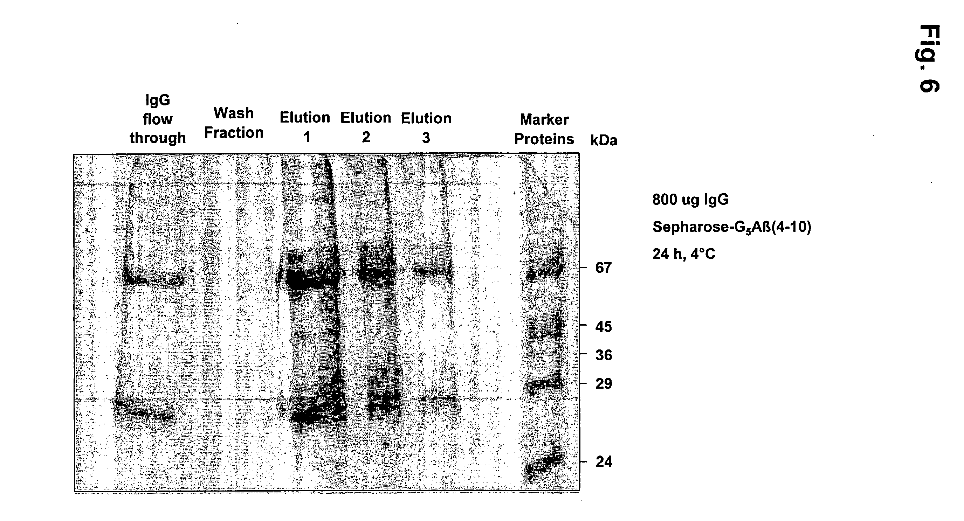

Figure 6: Isolation of "plaque-specific" antibodies recognising the N-terminal Aβ(4-10) epitope from the serum Aβ-autoantibodies of an Alzheimer patient, isolated by Aβ(4-10)-epitope specific chromatography. IgG stands for immunoglobulin G. AD signifies Alzheimer's Disease. Affinity column: G5Aβ(4-1 0). kDa: Molecular weight in kilodalton.

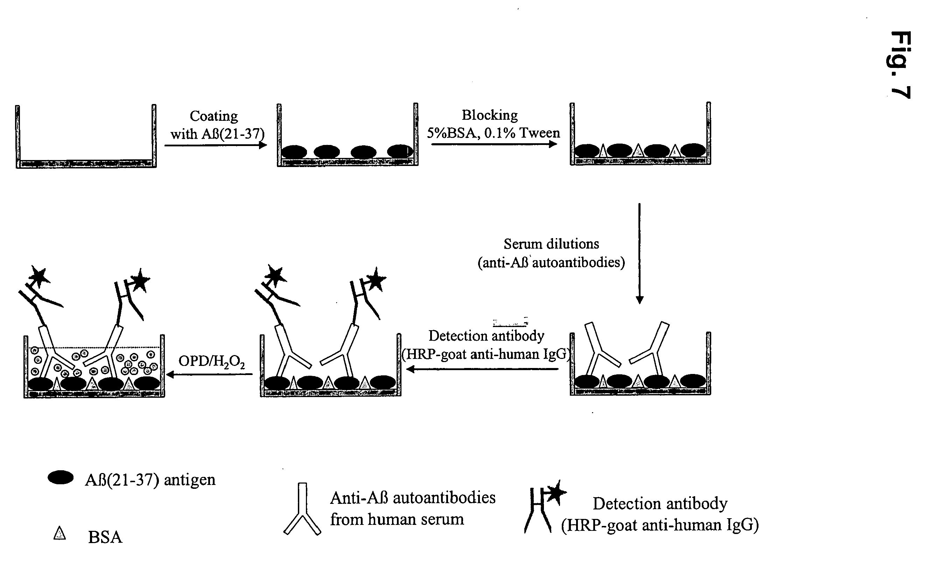

Figure 7: Serum ELISA for determination of anti-Aβ(21-37) autoantibodies. BSA is bovine serum albumine. HRP is horseradish peroxidase. OPD is o-Phenylenediamine. IgG stands for immunoglobulin G.

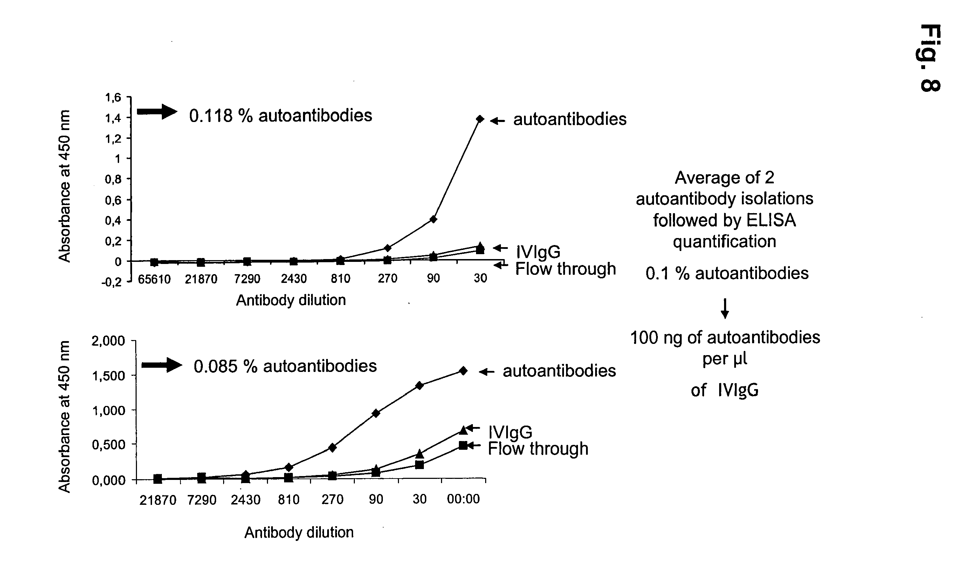

Figure 8: ELISA determination of Aβ-autoantoantibody (from IVIgG). IVIgG stands for intravenous IgG preparation. The ELISA was carried out with Aβ(1-40) coated on 96-well plate, and dilutions of Aβ-antibody were added, and determined with anti-human horseradish peroxidase as secondary antibody. Aβ-antibody quantifications were performed with a 1 µg/µl stock solution, using a BSA reference curve for calibration. The percentage indicated represents the Aβ-antibody concentrations in IVIgG from two separate ELISA determinations.

### Definitions

The term "polypeptide", as used herein, refers to a polypeptide chain of at least 5 amino acid residues. The term also refers to an assembly of more than one polypeptide chain, e.g. an assembly of four polypeptide chains, such as an antibody.

The term "polypeptide according to the present invention", as used herein, refers to a polypeptide comprising an amino acid sequence of an Aβ peptide, wherein the Aβ peptide has at least the sequence according to SEQ ID NO: 2, i.e. Aβ(21-37) and at most the sequence according to SEQ ID NO: 4, i.e. Aβ(12-40).

The term "autoantibody" or "autoantibodies", as used herein, refers to antibodies which are directed against epitopes on proteins of the human body and which can be found in the blood or cerebrospinal fluid of a human subject without prior immunisation with the respective antigen. The term herein particularly refers to antibodies specifically binding to the C-terminal half of Aβ polypeptide, in particular to Aβ(21-37)(SEQ ID NO:2). Preferably, such binding is specific, in particular under physiological conditions, e.g. pH about 7.4, salt concentrations about 50 to about 150 mM in PBS. Preferably, the specific binding has under in vitro conditions a relative dissociation constant (relative K_{D}; reflecting *in vitro* results but not necessarily identical values under *in vivo* conditions) of at least about 10⁻⁵ M or lower, more preferably about 10⁻⁶ M, about 10⁻⁷ M, about 10⁻⁸, about 10⁻⁹ M, about 10⁻¹⁰ M, about 10⁻¹¹ M, about 10⁻¹² M, or even less. In a particularly preferred embodiment the relative dissociation constant of the binding of said polypeptide to said epitope is in between about 10⁻⁸ M to about 10⁻¹² M, in particular around 1 to 50 x 10⁻⁹ M.

The term "epitope" or "epitope peptide", as used herein, generally refers to a polypeptide comprising the molecular recognition peptide sequence or structure derived from an antigen that is bound by a specific antibody. It is an immunological determinant group of an antigen which is specifically recognized by the antibody. An epitope may comprise at least 5, preferably at least 8 amino acids in a spatial or discontinuous conformation. An epitope may also comprise a single segment of a polypeptide chain comprising a continuous linear amino acid sequence with a minimal length of approx. 5 amino acids.

The term "plaque-specific" antibody, as used herein, refers to an antibody directed against the Aβ(4-10) epitope of Aβ.

The terms "neurodementing diseases", "dementing disorders" or "neurodementia diseases of AD-type", as used herein, refer to diseases selected from Alzheimer's disease, Down's syndrome, Dementia with Lewy bodies, fronto-temporal dementia as well as to disorders such as cerebral amyloid angiopathy and amyloidoses.

The term "moiety", as used herein, refers to a portion of a polypeptide with distinct function(s). Such moieties can provide for a structural or functional feature which is normally not present in the rest of the polypeptide or which feature is enhanced by this moiety. Such functional or structural moieties can for example provide binding, stabilisation or detection of the polypeptide. The moiety can be a polypeptide on its own or can be any other compound, which provides the desired function(s) to the polypeptide. Said moiety is stably associated with the polypeptide, in particular covalently coupled to the polypeptide. The term moiety, as used herein, does not confer any information about the size of this portion in comparison to the polypeptide itself. The moiety can be smaller, equally sized or larger than the polypeptide it is coupled to.

### Summary of the invention

In one aspect the present invention relates to a polypeptide comprising an amino acid sequence of an Aβ peptide, wherein the Aβ peptide has at least the sequence according to SEQ ID NO: 2 and at most the sequence according to SEQ ID NO: 4.

The polypeptide according to the invention thus comprises at least a sequence stretch identical to the Aβ peptide amino acid sequence ranging from amino acid 21 to amino acid 37 of SEQ ID NO: 1. This peptide sequence is denoted herein as SEQ ID NO: 2 or Aβ(21-37), respectively. A polypeptide according to the present invention can exhibit also longer sequence stretches of the Aβ peptide sequence, going beyond the Aβ(21-37) sequence. However, the length of the Aβ peptide sequence stretch comprised by the polypeptide according to the invention should not range further than from amino acid 12 to amino acid 40 of the Aβ peptide, also denoted herein as SEQ ID NO:4 or Aβ (12-40). This ensures, that the Aβ sequence stretch of the polypeptide of the invention does not comprise amino acids relevant for the binding of the plaque specific Aβ antibody found in patients with AD. The polypeptide according to the present invention can also comprise other amino acid sequences. For example, tags, markers, binding domains, activation domains or similar functional moieties can be fused to the Aβ sequence stretch, for instance, to provide for a better binding of the polypeptide according to the invention to certain surfaces. Analogous, the polypeptide according to the present invention can be modified for certain purposes, such as covalent coupling to a fluorophor or chromophor, biotin, or the like. In certain cases the non-Aβ sequence stretches of the polypeptide according to the invention provide for a structural stabilisation of the a Aβ(21-37) sequence stretch, preventing or reducing the formation of β-sheet conformation in this region.

In a preferred embodiment the above mentioned polypeptide additionally comprises one or more moieties such as tags or markers, in particular biotin, streptavidin, GST, HIS, STREP-tag, Myc, HA, poly-L-lysine, poly-L-lysine-L-alanine copolymers, poly-Aib (alpha-aminoisobutyric acid), poly-β-alanine, poly-L-alanine, poly-D-lysine, poly-D-lysine-D-alanine copolymers, poly-D-alanine, or combinations of poly-L- and -D-amino acids.

In a preferred embodiment the region of the above mentioned polypeptide, which has the sequence according to SEQ ID NO: 2 or any other sequence comprising Aβ(21-37) is not in β-sheet conformation. In an even more preferred embodiment this region is in random coil or exhibits α-helix conformation.

In another preferred embodiment the region of the above mentioned polypeptide having e.g. the sequence according to SEQ ID NO: 2 is flanked by amino acid sequences, which prevent or reduce β-sheet formation of the Aβ polypeptide region having e.g. the sequence according to SEQ ID NO: 2, in particular wherein said flanking amino acid sequences are located in close proximity to the N- and/or C-terminal ends of the Aβ sequence and which flanking amino acid sequences are composed of oligomeric peptides comprising, for example, L-alanine, D-alanine, Aib (alpha-aminoisobutyric acid), β-alanine, D-valine, L-glycine, D-glycine and/or related hydrophobic amino acids. Particularly preferred as flanking amino acid sequences are oligomeric peptides such as -(L-alanine)ₙ-, -(D-Alanine)ₙ-, -(Aib)ₙ- , -(β-alanine)ₙ₋, -(D-valine)ₙ₋, -(L-glycine)ₙ₋, -(D-glycine)ₙ₋ wherein n ranges preferably from about 2 to about 6.

Furthermore, the present invention relates to the use of a polypeptide according to the present invention and/or of a polypeptide comprising an amino acid sequence of an Aβ peptide, wherein the Aβ peptide has at least the sequence according to SEQ ID NO: 3 and at most the sequence according to SEQ ID NO: 5, for diagnostic assays. Methods for such diagnostic assays are exemplified below.

In another aspect the present invention relates to a method for diagnosing a neurodementing disease, the method comprising the following steps:
a) incubating a polypeptide according to the present invention immobilized on a carrier with a sample derived from a subject, subsequently
b) separating said sample from the carrier, and
c) detecting polypeptides bound to the immobilized polypeptide of step a).

The present invention also relates to a method for diagnosing a neurodementing disease, the method comprising the following steps:
a) incubating a polypeptide according to the present invention with a sample derived from a subject, and subsequently
b) incubating the sample with a carrier having a binding affinity for the polypeptide according to the present invention,
c) separating said sample from the carrier, and
d) detecting polypeptides bound to the polypeptide according to the present invention, said polypeptide being bound to the carrier.

In another aspect the present invention relates to methods of diagnosis of a neurodementing disease, which utilize a polypeptide or protein which comprises at least a sequence stretch identical to the Aβ peptide amino acid sequence ranging from amino acid 4 to amino acid 10 of SEQ ID NO: 1. This peptide sequence is denoted herein as SEQ ID NO: 3 or Aβ(4-10) or the N-terminal peptide, respectively. Such a polypeptide can exhibit also longer sequence stretches of the Aβ peptide sequence, going beyond the Aβ(4-10) sequence. However, the length of the Aβ peptide sequence stretch comprised by such a polypeptide should preferably not range further than from amino acid 1 to amino acid 20 of the Aβ peptide, also denoted herein as SEQ ID NO:5 or Aβ (1-20). This ensures, that the Aβ sequence stretch of the polypeptide of the invention does not comprise amino acids relevant for the binding of the Aβ autoantibody found in healthy individuals and directed to Aβ(21-37). The N-terminal polypeptide can comprise besides the Aβ sequence stretch other amino acid sequences, moieties and modifications as well, as already mentioned for the polypeptide according to the invention.

Thus, the present invention also relates to a method for diagnosing a neurodementing disease, the method comprising the following steps:
a) incubating a polypeptide immobilized on a carrier with a sample derived from a subject, wherein the polypeptide comprises an amino acid sequence of an Aβ peptide, wherein the Aβ peptide has at least the sequence according to SEQ ID NO: 3 and at most the sequence according to SEQ ID NO: 5, subsequently
b) separating said sample from the carrier, and
c) detecting polypeptides bound to the immobilized polypeptide of step a).

The present invention also relates to a method for diagnosing a neurodementing disease, the method comprising the following steps:
a) incubating a polypeptide with a sample derived from a subject, wherein the polypeptide comprises an amino acid sequence of an Aβ peptide, wherein the Aβ peptide has at least the sequence according to SEQ ID NO: 3 and at most the sequence according to SEQ ID NO: 5, and subsequently
b) incubating the sample with a carrier having a binding affinity for the polypeptide comprising an amino acid sequence of an Aβ peptide, wherein the Aβ peptide has at least the sequence according to SEQ ID NO: 3 and at most the sequence according to SEQ ID NO: 5,
c) separating said sample from the carrier, and
d) detecting polypeptides bound to the polypeptide, which comprises an amino acid sequence of an Aβ peptide, wherein the Aβ peptide has at least the sequence according to SEQ ID NO: 3 and at most the sequence according to SEQ ID NO: 5, said polypeptide being bound to the carrier.

In a preferred embodiment detection of polypeptides in step c) and d), respectively, is performed by means of ELISA, ELISPOT, Western-Blot, Dot Blot, Protein-Chip, surface plasmon resonance assay, immunoprecipitation or co-immunoprecipitation, or affinity chromatography, in particular immunoaffinity chromatography.

The present invention also relates to a method for diagnosing a neurodementing disease, the method comprising the following steps:
a) incubating a polypeptide according to the present invention immobilized on a carrier with a cell containing sample derived from a subject,
b) separating said sample from the carrier, and
c) detecting cells bound to the immobilized polypeptide of step a).

The present invention also relates to a method for diagnosing a neurodementing disease, the method comprising the following steps:
a) incubating a polypeptide according to the present invention with a cell containing sample derived from a subject, and subsequently
b) incubating the sample with a carrier having a binding affinity for a polypeptide of the present invention,
c) separating said sample from the carrier, and
d) detecting cells bound to the polypeptide according to the present invention, said polypeptide being bound to the carrier.

The present invention also relates to a method for diagnosing a neurodementing disease, the method comprising the following steps:
a) incubating a polypeptide immobilized on a carrier with a cell containing sample derived from a subject, wherein the polypeptide comprises an amino acid sequence of an Aβ peptide, wherein the Aβ peptide has at least the sequence according to SEQ ID NO: 3 and at most the sequence according to SEQ ID NO: 5,
b) separating said sample from the carrier, and
c) detecting cells bound to the immobilized polypeptide of step a).

The present invention also relates to a method for diagnosing a neurodementing disease, the method comprising the following steps:
a) incubating a polypeptide with a cell containing sample derived from a subject, wherein the polypeptide comprises an amino acid sequence of an Aβ peptide, wherein the Aβ peptide has at least the sequence according to SEQ ID NO: 3 and at most the sequence according to SEQ ID NO: 5, and subsequently
b) incubating the sample with a carrier having a binding affinity for the polypeptide comprising an amino acid sequence of an Aβ peptide, wherein the Aβ peptide has at least the sequence according to SEQ ID NO: 3 and at most the sequence according to SEQ ID NO: 5,
c) separating said sample from the carrier, and
d) detecting cells bound to the polypeptide, which comprises an amino acid sequence of an Aβ peptide, wherein the Aβ peptide has at least the sequence according to SEQ ID NO: 3 and at most the sequence according to SEQ ID NO: 5, said polypeptide being bound to the carrier.

In a further embodiment of the invention the polypeptide, which comprises an amino acid sequence of an Aβ peptide, wherein the Aβ peptide has the sequence according to SEQ ID NO: 3 and at most the sequence according to SEQ ID NO: 5, can comprise, as mentioned above, additional moieties such as tags or markers, in particular biotin, streptavidin, GST, HIS, STREP-tag, Myc, HA, poly-L-lysine, poly-L-lysine-L-alanine copolymers, poly-Aib (alpha-aminoisobutyric acid), poly-β-alanine, poly-L-alanine, poly-D-lysine, poly-D-lysine-D-alanine copolymers, poly-D-alanine, or combinations of poly-L- and -D-amino acids. In a preferred embodiment, a polypeptide of the invention is identical to the polypeptide, which comprises an amino acid sequence of an Aβ peptide, wherein the Aβ peptide has the sequence according to SEQ ID NO: 3 and at most the sequence according to SEQ ID NO: 5, except for the sequence stretch covering the Aβ-sequence.

In a preferred embodiment of the methods of the invention employing a polypeptide according to the invention a solvent is present in the incubation step(s), which prevents or reduces β-sheet formation of the polypeptide region having e.g. the sequence according to SEQ ID NO: 2. Preferred solvents may be trifluoroethanol (TFE), hexafluoro-isopropanol or similar solvents to stabilise peptide conformations and to prevent or reduce β-sheet formation. Preferably, the solvent is present in an aqueous solution comprising for example 5-10 mM phosphate buffer and 150 mM NaCl. In an even more preferred embodiment the concentration of TFE, hexafluoro-isopropanol and the like in the aqueous solution ranges from about 1 to about 5%, preferably from about 1% to about 2%.

In a preferred embodiment the methods of the invention comprise an additional step, wherein at least one washing step is performed before the detecting step.

In a preferred embodiment of the methods according to the present invention, relating to the detection of cells, the methods are carried out in form of a affinity chromatography, in particular immunoaffinity chromatography.

The amount of polypeptides, e.g. antibodies, binding to a polypeptide according to the present invention, e.g. Aβ (21-37), in a sample of a subject is an indicator for the status of the subject with regard to the development and/or progression of AD. The higher the concentration of polypeptides directed against the Aβ (21-37) sequence stretch, the higher the protective capacity and the lower the risk of development and/or progression of AD. The amount of polypeptides, e.g. antibodies, directed against the Aβ (4-10) epitope, in a sample of a subject, is an indicator for the status of the subject with regard to the development and/or progression of AD as well. However, in this case the situation is vice versa. The higher the concentration of polypeptides directed against the Aβ (4-10) sequence stretch, the higher the risk of development and/or progression of AD. For diagnostic reasons, the methods of detection according to the present invention can thus be performed individually, but also in combination in order to provide for a more specific diagnosis.

Thus, in a further embodiment of the invention a method according to the invention employing a polypeptide according to the invention is carried out in combination with a method of the present invention employing a polypeptide comprising an amino acid sequence of an Aβ peptide, wherein the Aβ peptide has at least the sequence according to SEQ ID NO: 3 and at most the sequence according to SEQ ID NO: 5. The method employing a polypeptide of the present invention can be performed simultaneously, prior or after the second method.

It is also possible to diagnose the neurodementing disease by way of an indirect approach. A method according to the present invention - utilizing either a polypeptide according to the present invention or a polypeptide comprising an amino acid sequence of an Aβ peptide, wherein the Aβ peptide has at least the sequence according to SEQ ID NO: 3 and at most the sequence according to SEQ ID NO: 5 - is combined with a similar method, which only differs from the methods of the present invention by utilizing polypeptide comprising a polypeptide having the length of Aβ(1-40) or Aβ(1-42), i.e. full length Aβ peptide instead of the shorter Aβ sequence stretches utilized by the methods of the present invention. In such a scenario, the two methods are carried out independently from each other and the results of the methods are compared. To illustrate this concept, the following example is given:
1) The method utilizing the Aβ full length polypeptide sequence yields the amount of all polypeptides (or cells producing such polypeptides, respectively) in the sample directed against full length Aβ (Result A).
2) On the other hand a method of the present invention utilizing a polypeptide of the present invention yields the amount of all polypeptides (or cells producing such polypeptides, respectively) in the sample directed against a polypeptide of the present invention such as Aβ(21-37) (Result B).
3) A person skilled in the art can now easily deduce the amount of polypeptides in the sample directed against an epitope at the N-terminus of Aβ, in particular against epitope Aβ(4-10), by subtracting Result B from Result A.

Analogous, the amount of polypeptides (or cells producing such polypeptides, respectively) in the sample directed against a polypeptide of the present invention such as Aβ(21-37) can be obtained by subtracting the results obtained with a polypeptide comprising an amino acid sequence of an Aβ peptide, wherein the Aβ peptide has at least the sequence according to SEQ ID NO: 3 and at most the sequence according to SEQ ID NO: 5, e.g. Aβ(4-10), from the results obtained with Aβ full length.

Thus, the present invention also refers to a method for diagnosing a neurodementing disease, wherein the methods according to the present invention comprise the following steps:
i) performing a first method according to the present invention as set forth above,
ii) performing a second method according to the present invention proviso that the polypeptide to be incubated in step a) of said second method comprises the full length amino acid sequence of Aβ peptide, and
iii) comparing the result obtained from step i) with the result of step ii).

A person skilled in the art will understand that it does not matter for the above method whether step i) is carried out prior, simultaneously with or after step ii).

In one embodiment the polypeptides to be detected in the methods of the invention are antibodies, in particular an Aβ(21-37) autoantibody or an Aβ(4-10) autoantibody.

In a preferred embodiment the methods according to the invention are carried out for diagnosing Alzheimer's disease, Down's syndrome, Dementia with Lewy bodies, fronto-temporal dementia, cerebral amyloid angiopathy, and/or amyloidoses.

In a further aspect the present inventions relates to a carrier comprising a polypeptide according to the invention. In a preferred embodiment the carrier additionally comprises a second polypeptide comprising an amino acid sequence of an Aβ peptide, wherein the Aβ peptide of the second polypeptide has at least the sequence according to SEQ ID NO: 3 and at most the sequence according to SEQ ID NO: 5.

The carriers according to the invention and used in the methods of the invention can be of any suitable material capable of binding polypeptides such as beads, in particular magnetic beads or sepharose beads, membranes, in particular polyvinylidene fluoride or nitrocellulose membranes, glass, sepharose matrices, gold surfaces, synthetic surfaces, in particular microtiter plates. For certain embodiments, the surface of the carriers can be coated with agents, which are, for instance, capable of binding to the tags and markers mentioned above.

In another aspect the present invention relates to a kit for the diagnosis of a neurodementing disease, wherein the kit comprises a polypeptide according to the invention.

In a preferred embodiment the above mentioned kit comprises a second polypeptide comprising an amino acid sequence of an Aβ peptide, wherein the Aβ peptide of the second polypeptide has at least the sequence according to SEQ ID NO: 3 and at most the sequence according to SEQ ID NO: 5. In an even more preferred embodiment the kit comprises a carrier, in particular a carrier as mentioned above.

In a preferred embodiment the sample or the cell containing sample, respectively, used for the methods of the present invention is derived from blood, plasma, urine or cerebrospinal fluid (CSF) of a subject. In an even more preferred embodiment the cell containing sample is derived from blood and the cells are of the B-cell lineage. The sample, i.e. the subject can be of human, rodent, bovine, porcine, canine or avian origin. In particular the sample or the cell containing sample can be derived from human, mouse, rat, rabbit, cow, pig, dog, chicken and so forth.

In this invention, the inventors identified two Aβ epitope sequences recognized by Aβ-autoantibodies isolated from serum of AD patients as well as of healthy control individuals. The Aβ-autoantibodies of healthy control individuals were found to specifically recognize the C-terminal part of the Aβ sequence, namely Aβ(21-37) (SEQ ID NO: 2). Furthermore AD patients have an increased fraction of antibodies recognizing the N-terminus, in particular the Aβ(4-10) epitope of the Aβ polypeptide (SEQ ID NO: 3), while having a decreased fraction of the antibodies recognizing the C-Terminus, in particular the Aβ(21-37) epitope of Aβ polypeptide. This provides the basis for a new, early AD diagnostic method by determination of Aβ antibodies, wherein an elevated level of Aβ-autoantibody (Aβ21-37) is a positive indicator, i.e. "healthy", while an elevated level of "plaque specific" Aβ(4-10)-antibody is a negative indicator, i.e. "sick", with respect to the prognosis for disease progression in a subject.

In one aspect the present invention relates therefore to a polypeptide comprising an amino acid sequence of an Aβ peptide, wherein the Aβ peptide has at least the sequence according to SEQ ID NO: 2 and at most the sequence according to SEQ ID NO: 4.

A polypeptide according to the invention having a sequence of Aβ as set forth above may be for instance the Aβ(21-37) fragment of amyloid beta itself. Other possible embodiments could comprise for example Aβ(20-37), Aβ(12-37), Aβ(12-40) (SEQ ID NO: 4), Aβ(20-40), Aβ(21-40) and so forth. This polypeptide fragment can be joined to other moieties. This means that a polypeptide according to the invention can comprise besides the Aβ(21-37) portion other polypeptide sequences or non-polypeptide structures or portions. The moieties attached to the Aβ polypeptide fragments may facilitate the performance of the methods according to the present invention.

Thus, in a preferred embodiment the polypeptide according to the invention additionally comprises other moieties such as tags or markers, which facilitate in particular the attachment of the polypeptide to a carrier. In particular such tags can provide for the immobilisation of the polypeptide on a carrier coated with the respective antagonist. Examples for such moieties are biotin, streptavidin, GST, HIS, STREP-tag, Myc, HA, poly-L-lysine, poly-L-lysine-L-alanine copolymers, poly-Aib (alpha-aminoisobutyric acid), poly-β-alanine, poly-L-alanine, poly-D-lysine, poly-D-lysine-D-alanine copolymers, poly-D-alanine, or combinations of poly-L- and -D-amino acids. The polypeptide comprising a sequence according to SEQ ID NO 3 and at most the sequence according to SEQ ID NO: 5, as used in some of the methods of the invention, can also comprise such additional moieties.

The above mentioned peptide markers can be directly fused to the polypeptides having e.g. the sequence of SEQ ID NO 2 or 3, respectively. If a higher flexibility between the marker/tag and the e.g. Aβ(21-37) peptide is desired, linkers can be introduced. Such linkers can be for instance polyglycine or - alanine linkers. Biotin, a non-peptidic substance, can be covalently linked to a polypeptide of the present invention. A multitude of possible combinations of markers and carriers for the immobilisation of a polypeptide of the present invention is known from the prior art.

In a preferred embodiment the region of the above mentioned polypeptide having e.g. the sequence according to SEQ ID NO: 2 is not in β-sheet conformation. The β-pleated sheet conformation of Aβ has been shown to be responsible for neurotoxicity. Thus, Aβ(21-37) antibodies in a healthy individual recognize in particular the Aβ(21-37) region, if it is in random coil or α-helix conformation. In another preferred embodiment, therefore, the region of the above mentioned polypeptide having the sequence according to SEQ ID NO: 2 is flanked by amino acid sequences, which prevent or reduce β-sheet formation of the polypeptide region having the sequence according to SEQ ID NO: 2. Preferably said flanking amino acid sequences are located in close proximity to the N- and/or C-terminal ends of the Aβ sequence stretch, e.g. having the sequence according to SEQ ID NO: 2 and/or which flanking amino acid sequences are composed of oligomeric peptides comprising, for example, L-alanine, D-alanine, Aib (alpha-aminoisobutyric acid), β-alanine, D-valine, L-glycine, D-glycine and/or related hydrophobic amino acids. Particularly preferred as flanking amino acid sequences are oligomeric peptides such as -(L-alanine)n-, -(D-Alanine)ₙ-, -(Aib)ₙ- , -(β-alanine)ₙ-, -(D-valine)ₙ- , -(L-glycine)ₙ-, -(D-glycine)ₙ- wherein n ranges preferably from about 2 to about 6. Such flanking regions also ensure, that the epitope Aβ21-37) of the polypeptides of the invention is not present in β-sheet conformation and thus accessible to the Aβ(21-37) autoantibodies in the samples.

However, in a particular embodiment, the polypeptide according to the present invention is a minimal polypeptide that has no further sequences than the sequence of Aβ(20-37), Aβ(12-37), Aβ(12-40), Aβ(20-40) or Aβ(21-40).

However, it has to be understood, that other polypeptides than the ones mentioned above, which bind specifically to a first monoclonal antibody, which first antibody is capable of binding specifically to a sequence as denoted in SEQ ID NO 2, but which polypeptides do not bind specifically to a second monoclonal antibody, which second monoclonal antibody is capable of binding specifically to a sequence as denoted in SEQ ID NO 3, can also be used for practicing the methods of the present invention.

In a preferred embodiment the polypeptide according to the invention is directly synthesized by conventional polypeptide synthesis methods (see also Example 5). Similar approaches are suitable for generation of a polypeptide comprising an amino acid sequence of an Aβ peptide, wherein the Aβ peptide has at least the sequence according to SEQ ID NO: 3 and at most the sequence according to SEQ ID NO: 5.

Alternatively, the polypeptide according to the invention can also be obtained by in vitro translation or via a recombinant expression system. In order to express a polypeptide according to the invention a respective nucleic acid expression construct has to be generated. A person skilled in the art will clearly see several ways to construct appropriate expression system harbouring a nucleic acid sequence encoding for a polypeptide according to the invention. The construct is subsequently expressed in a suitable host cell and the polypeptide is isolated. For this purification step the above mentioned markers and/or tags can be used as well.

The inventors found in serum of AD patients an additional fraction of antibodies directed against a N-terminal epitope of Aβ peptide, which is not present, or only in low abundance, in healthy individuals. Thus, the detection of such antibodies is indicative for the diagnosis, stage and/or progression of AD. To distinguish between Aβ(21-37) and Aβ(4-10) antibodies, for such diagnosis methods and assays a polypeptide can be used comprising an amino acid sequence of an Aβ peptide, wherein the Aβ peptide has at least the sequence according to SEQ ID NO: 3 (Aβ(4-10)) and at most the sequence according to SEQ ID NO: 5 (Aβ(1-20)).

In a preferred embodiment, the methods according to the invention comprising the detection of polypeptides in step c) or d), respectively, represent an ELISA. In this case the carrier is for example a microtiter plate. The separation of sample and carrier is achieved by removing the sample liquid from the microtiter plate and/or by washing the microtiter plate after the incubation. Preferably, the bound polypeptide is in this scenario an antibody and this antibody can be detected for example via a secondary antibody, coupled with e.g. alkaline phosphatase (AP), and the addition of a substrate for AP resulting in the turn over of the substrate into, for example, a coloured compound detectable by an optical device. A person skilled in the art and familiar with ELISA techniques will know several variations of the ELISA concept, which can be applied to the methods of the present invention as well.

Alternatively, in another preferred embodiment, the methods according to the invention comprising the detection of polypeptides in step c) or d), respectively, represent an ELISPOT assay. In this case the carrier is for example a nitrocellulose plate. The incubation step of the carrier with the sample provides in this scenario enough time for a cell in the sample to produce sufficient amounts of antibody. The separation of sample and carrier is achieved by removing the sample liquid from the nitrocellulose plate and/or by washing the nitrocellulose plate after the incubation. Preferably, the bound polypeptide is an antibody and this antibody can be detected, for example via a secondary antibody, coupled with e.g. alkaline phosphatase (AP), and the addition of a substrate for AP, such as BCIP/NBT (Bromo-chloro-indoryl phosphate / Nitro Blue Tetrazolium) resulting in the turn over of the substrate into, for example, a deep purple stain detectable visually or by an optical device. A person skilled in the art and familiar with ELISPOT techniques will know several variations of the ELISPOT concept, which can be applied to the methods of the present invention as well.

In a further embodiment, the methods according to the invention comprising the detection of polypeptides in step c) or d), respectively, represent a Western Blot or Dot Blot assay. In this case the carrier is for example a nitrocellulose membrane. For the Western Blot, on the nitrocellulose membrane is either immobilized a polypeptide as used in step a) of the methods according to the present invention or a substance with binding affinity for a polypeptide as used in step a) of the methods according to the present invention. The nitrocellulose or similar membrane (PVDF etc.) itself can provide for the binding affinity to the polypeptide as used in step a) of the methods according to the present invention. The separation of sample and carrier is achieved by removing the sample liquid from the nitrocellulose membrane and/or by washing the nitrocellulose membrane after the incubation. Preferably, the bound polypeptide is an antibody and this antibody is for example detected via a labelled secondary antibody, e.g. coupled with horseradish peroxidase, and subsequent luminescent reaction and detection. A person skilled in the art and familiar with Western Blot/Dot blot techniques will know several variations of the Western Blot/Dot blot concept, which can be applied to the methods of the present invention as well.

In a further embodiment, the methods according to the invention comprising the detection of polypeptides in step c) or d), respectively, represent a Protein chip, i.e. protein microarray. In this case the carrier is for example a glass surface functionalized for binding proteins. The separation of sample and carrier is achieved by removing the sample liquid from the glass carrier and/or by washing the glass carrier after the incubation. Preferably, the bound polypeptide is an antibody and this antibody is detected via a labelled secondary antibody, coupled with e.g. a fluorescent dye, which can be detected by an optical device. A person skilled in the art and familiar with protein chip techniques will know several variations of the protein chip concept, which can be applied to the methods of the present invention as well.

In a further embodiment, the methods according to the invention comprising the detection of polypeptides in step c) or d), respectively, represent a surface plasmon resonance analysis. In this case the carrier is for example a metal surface such as a gold surface. The separation of sample and carrier is achieved by removing the sample liquid from the metal carrier and/or by washing the metal carrier after the incubation. Preferably, the bound polypeptide is an antibody and the binding of the antibody is detected via measuring the intensity of the reflected light at a specific incident angle with an optical device. A person skilled in the art and familiar with plasmon resonance analysis techniques will know several variations of the surface plasmon resonance concept, which can be applied to the methods of the present invention as well.

In a further embodiment, the methods according to the invention comprising the detection of polypeptides in step c) or d), respectively, represent a pull down or immunoprecipitation experiment. In this case the carrier may consist of sepharose beads. This sepharose beads are coated for example with gluthation (for pull down assays) or with an antibody (immunoprecipitation assays). Separation of sample and carrier is achieved by removing the sample liquid from the sepharose beads and/or by washing the sepharose beads after the incubation. Preferably, the bound polypeptide is an antibody and this antibody is detected via a subsequent Western Blot analysis of the precipitated protein complexes. A person skilled in the art and familiar with Pull down/Immunoprecipitation techniques will know several variations of these concepts, which can be applied to the methods of the present invention as well. One such variation would be the application of a co-immunopreciptation approach, wherein the carrier has only an indirect binding affinity for the polypeptide as used in step a) of the methods according to the present invention.

In a further embodiment, the methods according to the invention comprising the detection of polypeptides in step c) or d), respectively, are carried out in form of an affinity chromatography. In this case the carrier is the matrix, e.g. sepharose within a conventional chromatography column, to which is either linked a polypeptide as used in step a) of the methods according to the present invention or a substance with binding affinity for a polypeptide as used in step a) of the methods according to the present invention. The incubation of the sample with the carrier comprises the time frame the sample needs to pass through the column. The separation of sample and carrier is achieved by eluting the sample liquid from the column and/or by washing the column after the incubation. Preferably, the bound polypeptide is an antibody and this antibody is for example detected by elution of the bound antibody, for instance with a buffer having a high salt content, and subsequent detection of eluted polypeptide, for example by direct optical determination or by subsequent Western blot or similar analyses. A person skilled in the art and familiar with affinity chromatography techniques will know several variations of the affinity chromatography concept, which can be applied to the methods of the present invention as well. One such variation would be the application of an immunoaffinity chromatography approach, wherein the carrier is coated with antibodies directed against a polypeptide as used in step a) of the methods according to the present invention.

In one embodiment, the methods according to the invention comprising the detection of cells in step c) or d), respectively, are carried out in form of an affinity chromatography. In this case, for example, magnetic beads coated with sepharose represent the carrier, to which either a polypeptide as used in step a) of the methods according to the present invention is linked or a substance with binding affinity for a polypeptide as used in step a) of the methods according to the present invention. The separation of sample and carrier is achieved by eluting the sample liquid from the column and/or by washing the column after the incubation. The cells bound to the matrix are for example B- or T-cells, which can be detected, after elution of the cells from the matrix, for example by way of flow cytometry. A person skilled in the art and familiar with affinity chromatography and flow cytometry techniques will know several variations of these concepts, which can be applied to the methods of the present invention as well.

Thus, the methods according to the present invention can be carried out in particularly preferred embodiments as ELISA, ELISPOT, Western-Blot, Protein-Chip, surface plasmon resonance assay, immunoprecipitation or co-immunoprecipitation, or affinity chromatography, in particular immunoaffinity chromatography. These are all exemplifications of diagnostic assays. Examples for ELISA's or affinity chromatography can be found in the examples section. In diagnostic procedures based on surface plasmon resonance (SPR) the detection, and quantification and analysis of binding kinetics of Aβ-epitope specific antibodies can be performed by binding of (i) a biotinylated Aβ(21-37)- or Aβ(4-10)-peptide to a avidin/streptavidin-coated SPR chip surface, or by binding (ii) Aβ(21-37)- or Aβ(4-10)-peptides with an N-terminal Thiol-group containing carboxylic acid spacer to a gold-chip surface; followed by binding and determination of the Aβ-autoantibodies. A person skilled in the art will readily know how to incorporate the methods according to the present invention into one of these standard techniques and procedures mentioned above.

It has to be understood, that although the methods according to the present invention can be carried out in form of one of the above mentioned detection techniques per se (ELISA, ELISPOT, Western-Blot, Dot Blot, Protein-Chip, surface plasmon resonance assay, immunoprecipitation, affinity chromatography, etc.), it is also possible to combine these detection techniques or to apply them only for the detection step according to the invention, i.e. step c) or d), respectively, while the other steps are carried out in other formats. It is also obvious to a person skilled in the art, that the information/signals obtained in the detection steps in the methods of the present invention can provide the basis for quantification of this information/signals.

In some cases it might be of higher diagnostic value, if, instead of or in addition to the detection of polypeptides, e.g. antibodies, cells producing said polypeptides are detected. For example, it could be of importance, if in an AD patient the overall number of cells producing an Aβ(21-37) autoantibody is lower than in healthy individuals, or if the amount of antibody secreted by the respective antibody producing cells is reduced. Depending on the result this can lead to different therapeutic approaches. Thus, the present invention also relates to the detection of cells producing polypeptides binding to a polypeptide according to the invention or binding to a polypeptide comprising an amino acid sequence of an Aβ peptide, wherein the Aβ peptide has at least the sequence according to SEQ ID NO: 3 and at most the sequence according to SEQ ID NO: 5.

As used in this invention, an immobilized polypeptide refers in this regard to a polypeptide, which is coupled to a carrier. The coupling can be covalently or non-covalently, it can be directly to the carrier or via a linker/linking substance. If the immobilization occurs non-covalently, then the carrier or the linking substance exhibits a specific binding affinity for the polypeptide according to the invention and vice versa. Binding affinity refers to a property of a substance, in particular a polypeptide, to associate with (an) other substance(s) and to form a stable specific dimeric or multimeric complex. Such associations rely usually on Van der Waals- or hydrogen-bonds.

The incubating step(s) serves the purpose two partners of a binding pair, i.e. having a binding affinity for each other, can associate and form a stable complex. The temperature of the incubation step may vary, but is usually from about 0°C to about 40°C, preferably from about 4 to about 37°C, even more preferred about 4°C, about 16°C, about 21°C or about 37°C. The higher the temperature, the shorter the time of incubation might be. For example, if the incubation temperature is 4°C it should last for at least 12h or over night, while 1h is usually enough for an incubation at 37°C. If suitable, the carrier can be blocked prior to the method with a suitable blocking agent, reducing the likelihood of unspecific binding events. Blocking agents can be for example milk powder, BSA, fetal calf sera, or any other blocking reagent.

The detection of polypeptides bound to an immobilized polypeptide of the invention or to a polypeptide comprising an amino acid sequence of an Aβ peptide, wherein the Aβ peptide has at least the sequence according to SEQ ID NO: 3 and at most the sequence according to SEQ ID NO: 5, can be performed by several means. One possibility would be for example the identification via mass spectrometrical means, for example MALDI-TOF, ESI-MS, MS-FTICR. To this purpose, immunoglobulins are first isolated from, for example, a serum sample of an AD patient by protein G affinity chromatography, and subsequently Aβ-autoantibodies and Aβ- plaque specific antibodies are e.g. isolated by Aβ-epitope-chromatography, respectively. The antibodies are then immobilised, for example, on a sepharose carrier as described in the examples. The specific Aβ -epitopes, Aβ(21-37) and Aβ (4-10), are then identified after binding of full-length-Aβ-polypeptide (for example Aβ(1-40) or Aβ(1-42) ), followed by proteolytic epitope-excision mass spectrometric analysis using one or several of the proteases, trypsin, chymotrypsin, Glu-C protease, Asp-N-protease. After washing the affinity-bound Aβ-epitope(s) until no signal is detected in the supernatant, the specific Aβ-epitope is eluted from the column by treatment with, typically, 0.1 % trifluoroacetic acid, and identified by accurate determination of its protonated molecular ions; the latter molecular ion mass accuracy is entirely sufficient for identification, but can be further ascertained by collision-induced fragmentation and tandem-MS analysis of fragment ions.

For certain embodiments, secondary antibodies labelled with a fluorescent dye or moiety (e.g. GFP) or labelled with an enzymatically active substance such as horseradish peroxidase, alkaline phosphatase, β-galactosidase or other related enzymes capable to convert a colourless substrate to a suitable dye or fluorescent product can be applied. The detection can also be accomplished by detecting the amount of occupied binding sites, i.e. utilizing a labelled Aβ(21-37) antibody, which is incubated with the carrier after the removal of the sample. The amount of bound Aβ(21-37) is in this scenario an indicator for the amount of prior bound polypeptide. The lower the amount of subsequently bound Aβ(21-37) antibody is, the more Aβ(21-37) binding polypeptides contained the sample. The mentioned examples of detection are not to be considered limiting, as a person skilled in the art will readily know a plurality of methods of detection which can be used in the present invention.

Usually, the polypeptides bound to the immobilized polypeptide, which are detected in step c) or d), respectively, in the methods of the present invention will be antibodies, in particular an Aβ(21-37) autoantibody or an Aβ(4-10) autoantibody, respectively. However, other substances in the human body may also bind for instance to Aβ(21-37) or Aβ(4-10) polypeptide.

Likewise, the detection of cells producing a polypeptide binding to a polypeptide of the present invention or binding to a polypeptide comprising an amino acid sequence of an Aβ peptide, wherein the Aβ peptide has at least the sequence according to SEQ ID NO: 3 and at most the sequence according to SEQ ID NO: 5, is accomplished by standard techniques known to a person skilled in the art. One example would be the analysis via flow cytometry. Another approach would be the lysis of the cells, DNA/RNA isolation and subsequent PCR amplification of specific nucleotide sequences. Besides this, in the prior art there are plurality of further possibilities published, which can be employed to detect the cells in the methods of the present invention.

A sample derived from a subject is derived from tissue or body fluid of a subject. The subject can be a healthy individual, i.e. not suffering from AD, or a "patient" suffering from a neurodementing disorder. In a preferred embodiment the sample or the cell containing sample, respectively, used for the methods of the present invention is obtained from blood, plasma, urine or cerebrospinal fluid (CSF) of a subject. In an even more preferred embodiment the cell containing sample is obtained from blood and the cells are of the B-cell lineage. The sample, i.e. the subject can be of human, rodent, bovine, porcine, canine or avian origin. In particular the sample or the cell containing sample can be derived from human, mouse, rat, rabbit, cow, pig, dog, chicken and so forth. Possible preparation procedures of such samples are well known from the prior art.

In a preferred embodiment of the methods of the present invention employing a polypeptide of the present invention a solvent is present in the incubation step(s), which prevents or reduces β-sheet formation of the polypeptide region having the sequence according to SEQ ID NO: 2. As mentioned above, the β-pleated sheet conformation of Aβ has been shown to be responsible for neurotoxicity. Thus, Aβ(21-37) antibodies in a healthy individual recognize in particular the Aβ(21-37) region, if it is in random coil or α-helix conformation. Analogous to flanking amino acid sequences, solvents can influence the conformational state of the polypeptides of the invention. In particular, TFE, hexafluoro-isopropanol and so forth can be used in the methods of the present invention, for example in the incubation step, to prevent or reduce a β-sheet conformation of the important epitope Aβ(21-37), thus keeping it accessible to the Aβ(21-37) autoantibodies in healthy individuals. Preferably, TFE is present in a concentration ranging from about 1% to about 5 %, preferably about 1 to about 2%.

In a preferred embodiment the methods of the invention comprise an additional step, wherein at least one washing step with a washing solution is performed before the detecting step. A washing step can increase the specificity of the later detection signal and reduces background signals. Preferably, the washing solution is water. More preferably buffers like PBS or TBS are used to ensure a constant pH. The washing solution can contain small amounts of detergent to increase the specificity of the signal. If the specificity of the signal is low, the salt concentration or the concentration of the detergent can be increased in the washing solution.

In a further embodiment of the invention a method according to the invention employing a polypeptide of the invention, i.e. Aβ(21-37) polypeptide, is carried out in combination with a method of the present invention employing a polypeptide comprising an amino acid sequence of an Aβ peptide, wherein the Aβ peptide has at least the sequence according to SEQ ID NO: 3 and at most the sequence according to SEQ ID NO: 5, i.e. Aβ (4-10). The method employing a polypeptide of the present invention can be performed simultaneously, prior or after the second method. The comparison of the abundance of Aβ(21-37) specific polypeptides with the abundance of Aβ(4-10) specific polypeptides will provide for a more detailed assessment of the stage and progression of AD.

The detecting step in the methods of the present invention provides for the possibility to quantify the amount of polypeptides bound to Aβ(21-37) or Aβ(4-10). The determined values are a measure for the stage and progression of AD. For instance, a human subject can be considered healthy in regard to AD, if its serum contains about 1 to 100 ng/µl of Aβ(21-37) specific polypeptides and/or about 0 ng/µl (i.e. below the detection limit) of the Aβ(4-10) specific polypeptides. As reference for a healthy individual might serve the average concentrations of the respective polypeptides in the serum of people in the age of 20 to 35. In contrast, a subject might suffer from a neurodementing disease or be endangered to develop a neurodementing disease, for instance, if its serum contains about 0.01 to 5 ng/µl of Aβ(4-10) specific polypeptides, preferably about 0.05 to 1 ng/µl or even more preferably about 0.01 ng/µl or if the ratio of the concentration of the plaque specific polypeptide vs. the concentration of the Aβ(21-37) specific polypeptides in the serum raises above 0, preferably if it is higher than 0.001, 0.002, 0.003, 0.004, 0.005, 0.010, 0.015, 0.020, 0.030 or even higher than 0.050. With ageing the amount of immunoglobulin produced in a human body decreases naturally. Therefore, in particular cases it might be necessary to consider the age of the subject before the results obtained with the methods according to the present invention are evaluated. In particular a person about 20 to about 35 years of age might be considered healthy, if its, for instance, serum contains about 30 to 100 ng/µl or more of Aβ(21-37) specific polypeptide, while a subject about 70 to about 80 years of age can still be considered equally healthy with regard to AD with "only" 2 to 5 ng/µl of Aβ(21-37) specific polypeptide in its serum.

In a preferred embodiment the methods according to the invention are carried out for diagnosing a neurodementing disease, Alzheimer's disease, Down's syndrome, Dementia with Lewy bodies, fronto-temporal dementia, cerebral amyloid angiopathy, and/or amyloidoses. All diseases have in common, that the concentration of Aβ-autoantibody and Aβ-plaque specific antibody is affected by the respective disease as given above for AD.

In a further aspect the present inventions relates to a carrier comprising a polypeptide according to the invention. In a preferred embodiment the carrier additionally comprises a second polypeptide comprising an amino acid sequence of an Aβ peptide, wherein the Aβ peptide of the second polypeptide has at least the sequence according to SEQ ID NO: 3 and at most the sequence according to SEQ ID NO: 5.

The carriers according to the invention and used in the methods of the invention can be of any suitable material capable of binding polypeptides such as beads, in particular magnetic beads or sepharose beads, membranes, in particular polyvinylidene fluoride or nitrocellulose membranes, glass, sepharose matrices, gold surfaces, synthetic surfaces, in particular microtiter plates. For certain embodiments, the surface of the carriers can be coated with agents, which are, for instance, capable of binding to the tags and markers mentioned above. A person skilled in the art will readily know a broad variety of different carriers and possible coatings, which can be applied for the methods according to the invention.

In another aspect the present invention relates to a kit for the diagnosis of a neurodementing disease, wherein the kit comprises a polypeptide according to the invention. In a preferred embodiment the above mentioned kit comprises a second polypeptide comprising an amino acid sequence of an Aβ peptide, wherein the Aβ peptide of the second polypeptide has at least the sequence according to SEQ ID NO: 3 and at most the sequence according to SEQ ID NO: 5. In an even more preferred embodiment the kit comprises a carrier, in particular a carrier as mentioned above. Such kits could be used for example for routine diagnostics in hospitals and nursing homes, for example to monitor the progression of AD or to monitor the effectiveness of an AD therapy.

The following examples explain the invention but are not considered to be limiting. Unless indicated differently, molecular biological standard methods were used, as e.g., described by Sambrock and Russel, 2001, Molecular cloning: A Laboratory Manual, 3. edition, Cold Spring Harbor Laboratory Press, Cold Spring Harbor, New York.

### Examples

### Example 1: Isolation of the Aβ-autoantibodies from healthy individuals:

### a) via an Aβ(1-42) column

Aβ-autoantibody was isolated from commercially available IVIgG and from serum (healthy individuals) by affinity chromatography using both a commercially available N-(S-palmitoyl)-Cysteinyl-Aβ-(1-42) affinity column, and an Aβ(1-42) column containing an N-terminal penta-glycine spacer group, immobilized on NHS-activated sepharose as previously described in detail (Du et al., 2001; Korecka et al., 2004; Tian et al., 2005; McLaurin et al., 2002).

Electrophoretic separation and isolation by isoelectric focusing of the Aβ-autoantibody was carried out by ID- and 2D-SDS-PAGE. For two dimensional electrophoresis, anti-Aβ autoantibody samples purified from human serum samples and IVIgG were solubilised in a solution containing 7 M urea, 2 M thiourea, 4% CHAPS, 0.3% DTT, 2% Servalyt 3-10 and a trace of bromophenol blue. The immobilised pH gradient (IPG) strips (pH range 3-10 or 6-11 linear, 17 or 18 cm) were rehydrated overnight using in-gel rehydration method. Isoelectric focusing (IEF) was carried out for about 30 kVh at 20°C with a Multiphor II horizontal electrophoresis system (Amersham Pharmacia Biotech). The second-dimensional separation was carried out with a Bio-Rad Protean II xi cell vertical electrophoresis system using 12% SDS-PAGE gels of 1.5 mm thickness. After IEF, strips were equilibrated for 30 min in 6M urea, 30% glycerol, 2% w/v SDS, 0.05 M Tris-HCl (pH 8.8), 1% DTT and a trace of bromphenol blue, then for 30 min in the same solution except that DTT was replaced by 4.5% (w/v) iodoacetamide. Equilibrated strips were placed on the vertical gels and were overlayed with 1% agarose in SDS running buffer (25 mM Tris-HCl, 192 mM glycine and 0.1 % w/v SDS) and subjected to electrophoresis at 25 mA/gel for 30 min and 40 mA/gel until the tracking dye reached the anodic end of gels. After separation in SDS-PAGE gels, the proteins were visualised by silver staining or by sensitive colloidal Coomassie staining and scanned using a GS-710 Calibrated Imaging Densitometer (Bio-Rad).

PDQuest software from Bio-Rad (standard version according to Suppliers procedures) was employed in the present work for imaging and analysing 2-D gels. Once the gel images were scanned using a red filter and a scan resolution of 84.7×84.7 µm/pixel, the resulting image was corrected for background noise and streaking (vertical and horizontal) and then median-smoothed. Spots were detected using spot-detection wizard by setting the same parameters on all the gels. The spots were also inspected manually to remove obvious artefacts, such as speckles and lines along the edge of the gels before comparative spot analysis.

### b) via an Aβ(12-40) column

The Aβ-autoantibodies were isolated from (i), serum immunoglobulin (serum IVIgG; obtainable for instance from Octapharma GmbH, Germany) and (ii) from serum of healthy individuals (HI; see designations of samples in the Figures). Isolation of antibodies was performed by Aβ-epitope-specific affinity chromatography by a procedure that employed a N-cysteinyl- Aβ(12-40) column which was immobilized on Ultralink-iodoacetyl- solid phase carrier as described below.

N-Cysteinyl -Aβ(12-40) (H-CVHHQKLVFFAEDVGSNKGAIIGLMVGGW-COOH) was synthesized by solid phase peptide synthesis using 9-fluorenylmethoxycarbonyl/t-buthyl (Fmoc/tBu) chemistry on a NovaSyn TGR resin (0.23 mmole/g coupling capacity) on a semi-automated Peptide Synthesizer EPS-221 (INTAVIS, Langenfeld, Germany). The following side-chain protected amino acid derivatives were used: Fmoc-Lys(Boc)-OH, Fmoc-Asn(Trt)-OH, Fmoc-Ser(tBu)-OH, Fmoc-Asp(OtBu)-OH, Fmoc-Glu(OtBu)-OH, Fmoc-Gln(Trt)-OH, Fmoc-His(Trt)-OH, Fmoc-Cys(Trt)-OH. The synthesis was performed according to the following protocol: (i) DMF washing; (ii) Fmoc deprotection with 2 % DBU, 2 % piperidine in DMF (5 + 10 min), (iii) DMF washing, (iv) coupling of 5 equiv of Fmoc amino acid: PyBOP : NMM in DMF (40 min), (v) coupling of 5 mol-equivalents of Fmoc amino acid: PyBOP : NMM in DMF (40 min) (vi) DMF washing (3 x 1 min). Due to the hydrophobic character of the C-terminal sequence of Aβ, double coupling of each amino acid was employed throughout the synthesis. After completion of the synthesis cycles, the peptide was cleaved from the resin for 3 h using a mixture containing 95 % TFA, 2.5 % triisopropylsilan and 2.5 % deionized water. The crude product was precipitated with cold tert-butylmethylether, washed three times with diethyl ether and solubilized in 10% acetic acid (aqueous solution) prior to freeze-drying. Purification of the peptides was performed by semipreparative HPLC; subsequent characterisation by HPLC and MALDI-TOF mass spectrometric analysis ensured molecular homogeneity of the peptide.

*Immobilisation of CysAβ(12-40) on Ultralinklodoacetyl Gel.-* Since the Aβ (12-40) sequence contains two internal lysine residues which might lead to side reactions in immobilisation procedures using amino groups, a specific affinity column was prepared using a cysteine residue attached to the Aβ- N-terminus, to ensure homogeneous orientation of peptide molecules on the column support by immobilisation through cysteinyl-S-thioether linkage. The azlactone-activated support contains an iodoacetyl group (UltraLink; Perbio, Bonn, Germany) at the end of a hexadecyl- spacer group, which was reacted with the cysteinyl-sulfhydryl group to yield a stable thioether linkage, in order to reduce steric hindrance and provide maximum binding capacity of the antibodies. For covalent attachment of the Cys-Aβ(12-40), 3.7 mg of peptide were dissolved in 50 mM Tris, 5 mM EDTA-Na coupling buffer (pH 8.5) to a final concentration of 0.37 mg/ml. The solution was added to 1 ml of drained Ultralink- Iodoacetyl Gel and the coupling reaction was performed for 1 hr at 25°C under gentle mixing, followed by 30 min reaction time without mixing. An aliquot of 0.5 ml of the Cys-Aβ(12-40) coupled support was packed into a column (2.5 ml, MoBiTec, Göttingen, Germany) allowing the solution to drain. The column was washed with 3 ml of coupling buffer, and non-specific binding sites on the gel were blocked for 2 x 45 min by reaction with 1 ml of 50 mM L-Cysteine-HCI in coupling buffer. Subsequently the column was washed with 5 ml of 1 M NaCl and 5 ml of 0.1 M Na-phosphate, 0.15 M NaCl (pH 7.2) and stored at 4°C. The gel support (0.5 ml) was transferred into a 15 ml Falcon vial using 5 ml PBS and mixed with 5 ml IVIgG. After gentle shaking overnight at 4 °C, the suspension was transferred to the column using the effluent to completely rinse the matrix back into the column. The column was washed eight times with 10 ml of PBS followed by 2 wash cycles with 10 ml ultrapure water. The affinity-bound antibodies were eluted from the column with 10 x 0.5 ml 0.1 % trifluoroacetic acid (TFA). Subsequent isolation and preparation of IgG for affinity studies was performed using the following protocol:

The procedure involved adjustment to neutral pH for each fraction collected using 0.5 M NaH₂PO₄ (pH 8) in order to maintain integrity of the antibodies for use in affinity studies. The bound antibodies were eluted from the column with 10 x 0.5 ml 0.1 M glycine buffer, pH 2.8. Each fraction was collected in a microreaction tube containing 35 µl 1M Tris-HCl, pH 9. To maintain integrity of the antibodies neutral pH was adjusted immediately after elution by adding the appropriate amount of Tris-HCl or glycine buffer. To regenerate the column for further use, the column was washed once with 10 ml 10 mM sodium-phosphate buffer pH 6.8, followed by two wash cycles with 10 ml of PBS containing 1M sodium chloride and finally two wash cycles with 10 ml PBS. Protein concentrations were determined by the BCA method (Pierce; Perbio, Bonn, Germany).

*Antibody quantification.* Antibody concentrations in the elution fractions were determined by the Micro BCA™ Protein Assay Kit method (Pierce; Perbio, Bonn, Germany). The stock solution of 2 mg/ml of bovine albumin supplied within the Micro BCA^{™} Kit was used to prepare fresh standard dilutions within the range 40 - 0.5 µg/ml. The antibodies eluted between fractions 1 to 6, with highest concentrations in fractions 1 and 3. For quantification of each set of 10 elution fractions, fresh albumin standard dilutions were prepared. Results were read at 562 nm with the ELISA reader.

### Example 2: Isolation of Aβ-antibody from an AD patient

Immunoisolation of the serum Aβ-antibody from an AD patient by epitope-specific affinity-chromatography was performed on a Sepharose-G5Aβ(4-10) affinity matrix column. The Sepharose-GSAβ(4-10) affinity matrix was washed with 10 ml of PBS (5 mmol L⁻¹ Na₂HPO₄, 150 mmol L⁻¹ NaCl, pH 7.5) and transferred into a 1.7 ml vial using 300 µl of PBS. 800 µl (1 µg/µl) of two distinct autoantibodies (isolated from the sera of Alzheimer patients) were added and the sample was slowly rotated overnight at 4°C. The suspension was transferred to a 0.8 ml micro-column (Mobitec, Gottingen, Germany) providing the possibility of extensive washing without significant loss of material. The first 2 ml were collected as flow through fraction. The column was washed with 20 ml of PBS and the last 1 ml was collected for one-dimensional electrophoresis. The affinity bound IgG was eluted with 6× 0.5 ml 0.1 % TFA; the column was shaken gently for 15' and the released antibody molecules collected in a microreaction cup. The samples were lyophilised and stored until 1D-SDS-PAGE analysis.

Serum samples from healthy controls from all age ranges investigated were tested for the presence of plaque-antibodies (N-terminal epitope), using the Aβ(4-10) epitope column. In all investigated samples, non-AD control samples were devoid of detectable plaque-specific antibody.

### Example 3: Determination of the epitope recognized by the Aβ autoantibodies isolated from healthy individuals via Epitope excision

Antibody immobilisation was performed with a solution of 100 µg Aβ-IgG in 500 µl 0.2 M NaHC0₃/0.5 M NaCl (pH 8.3), which was added to n-hydroxysuccinimidyl (NHS)-activated 6-aminohexanoic acid-coupled sepharose (Sigma, St Louis, USA) and allowed to bind for 60 min at 20°C before transferring onto a microcapillary (MoBiTec, Goettingen, Germany). The column was washed two times alternately with blocking (ethanolamine/NaCl) and washing buffers (NaAc/NaCl). Epitope excision was performed by application of 2-5 µg Aβ-antigen and Aβ-antigen-containing material to the antibody microcolumn for 60 min at 20°C. After washing, digestion was performed on the column for 2 h at 37 °C with 0.2 µg protease in 200 µl PBS. Unbound peptides were removed and the epitope was dissociated from the antibody using 500 µl 0.1 % trifluoroacetic acid. After incubation for 15 min at 20°C, the epitope eluate was lyophilized and reconstituted in 10 µl 0.1% TFA for mass spectrometric analysis. Epitope extraction was performed in an analogous manner, however proteolytic digestion was performed first with the unbound antigen and the proteolytic digest was applied directly to the antibody column. Upon antibody complexation and epitope excision, relevant amino acid residues, e.g. F4, R5, D7, E11 (see Fig. 4), were found accessible to digestion while these residues are all shielded in the immune complex with the plaque-specific Aβ(4-10) antibody (s. Figure 3). In all non-AD control sera, the carboxy-terminal (Aβ21-37) sequence was found to be specifically recognised (proteolytically shielded), while N-terminal residues of Aβ were accessible for cleavage. The extracted epitope bound by the Aβ-autoantibody isolated from the healthy individuals exhibited thus the amino acid sequence of Aβ(21-37).

The structure and conformational properties, binding affinity and specificity of the Aβ-autoantibody epitope were further characterised by investigation of synthetic peptides comprising the Aβ(21-37) epitope sequence, and by fine-structure mapping using alanine sequence mutations, H-D exchange and high resolution mass spectrometry, ELISA studies and CD spectroscopic conformational analysis in different solvents. Biotinylated Aβ(21-37) peptides and peptides derivative flanked with oligo-glycine and -(D-Ala) spacer groups were synthesized by solid-phase peptide synthesis according to previously described procedures for Aβ-peptides, and were purified by reversed-phase HPLC and characterised by MALDI- and ESI- mass spectrometry for molecular homogeneity (Manea et al., 2004; Mezo et al., 2004). Comparative binding studies were performed with Aβ-epitope peptides comprising different C-terminal sequence lengths, using an ELISA system. These results established an essential function for antibody affinity of the carboxyterminal sequence end of Aβ, comprising residues 30-37; this partial sequence is critically involved in β-sheet formation and aggregation of Aβ. Thus, full binding affinity is obtained in Aβ(20-37) peptide (K_{D} approx. 80 nmol), as well as in peptides with extended C-terminal sequences (e.g., Aβ(12-40). In contrast the shortened Aβ(20-30) peptide showed almost completely abolished affinity. Mass spectrometric studies of peptides upon H-D equilibrium exchange showed rapid deuterium incorporation of peptide backbone hydrogens only for the Aβ sequence (20-30), but only little backbone deuteration for residues (30-37), suggesting increased shielding in this part due to conformational or aggregation effects. Control binding studies of the epitope peptide Aβ(20-37) with antibodies that recognized the N-terminal, Aβ(1-16) peptide (plaque-specific mono- and polyclonal antibodies) did not show any binding affinity.

### Example 4: Mass spectrometry

Sample preparation for mass spectrometry analysis was as described above.

MALDI-FTICR-MS/MS analyses were performed with a Bruker ApexII FTICR spectrometer equipped with SORI-CID dissociation (sustained off resonance irradiation collision induced dissociation) and IRMPD (Infrared Multiphoton Photodissociation) instrumentation for fragmentation of peptide and protein ions (Damoc et al., 2003). Once ions have been formed (by either MALDI or ESI ionization methods) and trapped in the analyzer cell, isolation of a precursor ion was achieved by ejecting from the cell all ions of higher and lower masses through the application of suitable excitation pulses with the appropriate frequencies and amplitudes. Experimental conditions: correlated sweep attenuation: 8-10 dB, ejection safety belt: 500-1000 Hz.

For SORI-CID, a low-amplitude r.f. excitation was applied for 250 msec to the precursor ion at a frequency that is slightly off-resonance (500-1000 Hz) from the cyclotron frequency. The amplitude of the excitation was kept low so that the ion never went to far from the center of the cell. While this excitation was applied, the pressure was raised in the analyzer cell (10⁻⁸ mbar) by admitting a collision gas (argon) through a pulse valve for 20-80 msec. Under these conditions, the precursor ion underwent many low-energy collisions, which slowly activated the ion until it reached its threshold for dissociation.

For IRMPD experiments the mass-selected ions were photodissociated using a 25 W continuous wave CO₂ laser (10.6 µm, Synard, Mukilteo, WA, USA). The laser power was set to 50% and the laser irradiation time to 50-200 msec.

The following search engines were employed for protein identifications and sequence assignments:
**Mascot -** Peptide mass fingerprint and MS/MS ion search from Matrix Science Ltd., London: **ProFound -** Peptide mass fingerprint from Rockefeller and New York Universities: **MS-Fit -** Peptide mass fingerprint from University of California, San Francisco (UCSF): **MS-Tag -** MS/MS ion search from University of California, San Francisco (UCSF):

### Example 5: Peptide synthesis

Peptides Biotin-G₅-FAEDVGSNKGA-NH₂ (Biotin-G₅-Aβ20-30) and Biotin-G₅-FAEDVGSNKGAIIGLMVG-NH₂ (Biotin-G₅-Aβ20-37) were synthesized by solid-phase peptide synthesis (SPPS) on a NovaSyn TGR resin, containing a polystyrene-polyethyleneglycol resin and Rink-amide- linker cleavable under acidic conditions, according to commercially available material and published literature procedures. 9-Fluorenylmethoxycarbonyl/t-butyl (Fmoc/tBu) chemistry was used throughout for synthesis using a semi-automated Economy Peptide Synthesizer EPS-221 (ABIMED, Germany). The following side-chain protected amino acid derivatives were used: Fmoc-Lys(Boc)-OH, Fmoc-Asn(Trt)-OH, Fmoc-Ser(tBu)-OH, Fmoc-Asp(OtBu)-OH, Fmoc-Glu(OtBu)-OH. The synthesis was performed according to the following general protocol: (i) DMF washing (3×1 min), (ii) Fmoc deprotection with 2%DBU, 2% piperidine in DMF (15 min), (iii) DMF washing (6×1 min), (iv) coupling of 5 equiv of Fmoc amino acid : PyBOP : NMM in DMF (40-60 min), (v) DMF washing (3×1 min). For the synthesis of Aβ (20-37) which has a hydrophobic C-terminal part, double coupling of each amino acid was employed. The biotinylation of the N-terminus was carried out on the resin using D-(+)-Biotin. After completion of the syntheses, the peptides were cleaved from the resin using a TFA, triethylsilane and deionized water mixture (95:2.5:2.5, V/V/V) for 3 h at room temperature. The synthetic peptides were used for example in ELISAs.

### Example 6: Amino acid sequencing of Aβ(21-37)

Sequence determination of all epitopes either identified (isolated Aβ epitopes Aβ(4-10) and Aβ(21-37)) or used (e.g. synthetic Aβ(21-37)) was carried out by means of
a) Edman sequencing;
b) ESI- Tandem MS/MS- sequencing, and
c) FTICR-MS analysis und fragmentation by means of IRMPD-Fragmentation.

Automated amino acid sequence analysis was performed on an Applied Biosystems Model 494 Procise Sequencer attached to a Model 140C Microgradient System, a 785A Programmable Absorbance Detector and a 610A Data Analysis System.

All solvents and reagents used were of highest analytical grade purity (Applied Biosystems). The sequencing method used was pulsed liquid. Lyophilised samples were dissolved in 10 µL 0.1 % TFA. To assure a distribution as close to the centre of the glass fibre filter as possible, the sample was applied in aliquots of 2 µL, each application followed by drying under a stream of argon.

### Example 7: ELISAs

ELISAs were used for the determination of:
(a) plaque-specific anti-Aβ(4-10) antibodies in the anti-Aβ autoantibodies mixture separated from IvIgG
(b) binding of anti-Aβ autoantibodies from human serum to Aβ(21-37) peptide, Aβ(12-40) peptide, and Aβ(1-40) peptide
(c) binding of anti-Aβ autoantibodies separated from IvIgG and from individual human sera (AD serum and healthy individuals serum) to Aβ(1-40) peptide and to Aβ(21-37) epitope peptide
(d) binding of anti-Aβ autoantibodies separated from IvIgG to Aβ partial sequences.

### a) ELISA for Aβ(4-10) antibodies

In this experiment a standard dilution of the antibody (anti-Aβ antibodies isolated from IvIgG using a Cys-Aβ(1-40) antigen column) was used in combination with 12 serial dilutions of Biotin-G₅Aβ(4-10) peptide, used as coating antigen. 96-well ELISA plates were coated with 150 µL/well streptavidin solution (c=5 µg/mL in PBS) for 2 hours at room temperature. After washing the wells four times with PBS-T (0.05% Tween-20 v/v in PBS, pH=7.5), 100 µL/well of biotinylated epitope peptides (12 serial dilutions from 50 µM to 0.024 µM in PBS, pH = 7.5) was added and incubated for 2 hours at room temperature. After that, the plates were washed four times with 200 µL/well PBS-T and the non-specific adsorption sites were blocked with 5% BSA, 0.05% Tween-20 in PBS (200 µL/well, 2 h incubation at RT). Then, 100 µL/well of the anti-Aβ autoantibodies isolated from IvIgG (1:150 dilution prepared in 5% BSA, 0.05% Tween-20 in PBS) was added to each well. Thereafter, the plates were incubated at room temperature for two hours and subsequently washed six times with PBS-T. 100 µL of peroxidase goat anti-human IgG diluted 5000 times in 5% BSA, 0.05% Tween-20 were added to each well and the plates were incubated at room temperature for one hour, then they were washed three times with PBS-T and once with 0.05 M sodium phosphate-citrate buffer, pH=5. 100 µL of o-Phenylenediamine dihydrochloride (OPD) in substrate buffer (phosphate-citrate) at c=1 mg/mL with 2 µL of 30% hydrogen-peroxide per 10 mL of substrate buffer were added. The absorbance at 450 nm was measured on a Wallac 1420 Victor² ELISA Plate.

### b) Antibody determination in human serum by indirect ELISA

96-well ELISA plates were coated with 100 µL/well of Aβ1-40 peptide (c=2.5 µg/mL in PBS buffer, pH 7.5) for 2 h at room temperature. Thereafter, the plates were washed four times with 200 µL/well of washing buffer (PBS-T; PBS with 0.05% Tween-20) and blocked for 2 h at room temperature with blocking buffer (5% BSA, 0.1% Tween-20 in PBS). After two times washing with PBS-T, the sera were added at an initial dilution of 1:33.3, then diluted 3 fold serially in blocking buffer and incubated for 2 h at room temperature. Then, the plates were washed eight times with PBS-T and goat anti-human IgG conjugated with horseradish peroxidase diluted 1:5000 in blocking buffer was added to the plates and incubated for 1 h at RT. The plates were washed four times with PBS-T and two times with 0.05 M sodium phosphate-citrate buffer, pH=5. 100 µL of o-Phenylenediamine dihydrochloride (OPD) in substrate buffer (phosphate-citrate) at c=1 mg/mL with 2 µL of 30% hydrogen-peroxide per 10 mL of substrate buffer were added. The absorbance at 450 nm was measured on a Wallac 1420 Victor² ELISA Plate. Aβ-antibody quantifications were performed with a 1 µg/µl stock solution, using a BSA reference curve for calibration using the commercial protein quantification kit Pierce micro-BCA. The results obtained are illustrated in Figure 8. The percentage given illustrates the Aβ-antibody concentrations in IVIgG from two separate ELISA determinations. Similar results were obtained with the Aβ(21-37) affinity chromatography. To the contrary, affinity chromatography with Aβ(4-10) peptide yielded no detectable amounts of polypeptides binding to the N-terminal epitope. Consequently, the results obtained with Aβ(1-40) are equivalent to those obtained with Aβ(21-37) for healthy individuals.

### c) Binding of anti-Aβ autoantibodies isolated from IvIgG and from individual human serum to Aβ1-40 peptide

96-well ELISA plates were coated with 100 µL/well of Aβ1-40 peptide (c=2.5 µg/mL in PBS buffer, pH 7.5) for 2 h at room temperature. Thereafter, the plates were washed four times with 200 µL/well of washing buffer (PBS-T; PBS with 0.05% Tween-20) and blocked for 2 h at room temperature with blocking buffer (5% BSA in PBS). After washing the plates two times with 200 µL/well of PBS-T, 100 µL/well of the 1^{st} antibody (polyclonal anti-Aβ autoantibodies isolated from IvIgG or from individual human serum) (8 serial dilutions prepared in blocking buffer; dilutions from 1:250 to 1:32000) was added and incubated for 2 h at room temperature. Then, the plates were washed four times with 200 µL/well of PBS-T and the 2^{nd} antibody (HRP-goat anti-human IgG; c=1 µg/µL) diluted 2000 times in blocking buffer was added (100 µL/well; 2 h incubation at room temperature). After washing the plates three times with 200 µL/well of PBS-T and once with 200 µL/well of citrate-phosphate buffer, pH=5, 100 µL of o-Phenylenediamine dihydrochloride (OPD) in substrate buffer (phosphate-citrate) at c=1 mg/mL with 2 µL of 30% hydrogen-peroxide per 10 mL of substrate buffer were added. The absorbance at 450 nm was measured on a Wallac 1420 Victor² ELISA Plate.

### d) Binding of anti-Aβ autoantibodies isolated from ivIgG to Aβ(21-37) epitope and other Aβ partial sequences

Peptides Biotin-G₅-Aβ(12-40), Biotin-G₅-Aβ(21-37), Biotin-G₅-Aβ(20-30), Biotin-G₅-Aβ(4-10) were compared for binding to anti-Aβ autoantibodies isolated from IvIgG by the following indirect ELISA: 96-well ELISA plates were coated with 150 µL/well streptavidin solution (c=5 µg/mL in PBS) for 2 hours at room temperature. After washing the wells four times with PBS-T (0.05% Tween-20 v/v in PBS, pH=7.5), 100 µL/well of biotinylated epitope peptides (12 serial dilutions from 50 µM to 0.024 µM in PBS, pH = 7.5) was added and incubated for 2 hours at room temperature. After that, the plates were washed four times with 200 µL/well PBS-T and the non-specific adsorption sites were blocked with 5% BSA, 0.05% Tween-20 in PBS (200 µL/well, 2 h incubation at RT). Then, 100 µL/well of the anti-Aβ autoantibodies isolated from IvIgG (1:150 dilution prepared in 5% BSA, 0.05% Tween-20 in PBS) was added to each well. Thereafter, the plates were incubated at room temperature for two hours and subsequently washed six times with PBS-T. 100 µL of peroxidase goat anti-human IgG diluted 5000 times in 5% BSA, 0.05% Tween-20 were added to each well and the plates were incubated at room temperature for one hour, then they were washed three times with PBS-T and once with 0.05 M sodium phosphate-citrate buffer, pH=5. 100 µL of o-Phenylenediamine dihydrochloride (OPD) in substrate buffer (phosphate-citrate) at c=1 mg/mL with 2 µL of 30% hydrogen-peroxide per 10 mL of substrate buffer were added. The absorbance at 450 nm was measured on a Wallac 1420 Victor² ELISA Plate.

### Example 8: Determination of dissociation constants of antigen-antibody-complexes by ELISA

The K_{d} values were determined by a modification of the method of Kim et al. (1990). For the determinations, the antibody concentrations employed were first derived from an initial calibration curve obtained by an indirect ELISA as described in Example 7b):
1) For the indirect ELISA, microtiter plates were coated with 150 µL/well of streptavidin at 20 °C for 2 hrs. Wells were washed one time with 0.05 % (v/v) Tween- 20 detergent in phosphate-buffered saline (PBS) (Na₂HPO₄ 5 mM, NaCl 150 mM, pH 7.5). Biotinylated-(G)₅-Aβ(12-40) peptide at concentrations between 1 × 10⁻⁶ and 10⁻⁸ M were prepared in PBS and deposited in the wells at a volume of 100 µL/well. The wells were incubated for 2 hours at 20 °C temperature followed by a 4 times washing step and blocking with blocking buffer (BSA 5 % w/v, 0.05 % Tween-20 v/v in PBS) for 2 hours. Anti-Aβ(12-40) antibody was diluted to concentrations between 1.4 × 10⁻⁷ and 10⁻⁹ M with blocking buffer and added at 100 µL/well. The microplate was incubated 20°C for 2 hours and then washed with Covabuffer (0.15 M PBS, pH 7.2 containing 2M NaCl, 0.083 M MgSO₄ and 0.05% Tween-20). The wells were incubated with peroxidase-conjugated mouse anti-human IgG (1:5.000) for 45 min at 20°C . Antibody binding was detected with a freshly prepared solution of 1,2-Phenylendiamine (OPD) containing 0.1 M citrate-phosphate, 0.1 % OPD and 0.006 % hydrogen peroxide. The enzyme reaction was monitored as a function of time at 450 nm, using an ELISA plate reader (Victor², Perkin Elmer Life/Analytical Sciences, Boston, MA). For each antibody and antigen concentration triplicate wells were prepared and measured. Direct proportionality was observed between absorbance and antibody concentration over a wide concentration range. This concentration range was used to select the initial concentration for K_{d} determinations. The initial concentration was selected to be within the linear region of the plot of optical density vs. antibody concentration.
2) For the determination of the K_{d} values the following conditions were applied. The antigen, Aβ(12-40) peptide at various concentrations (1 × 10⁻⁶ M to 4.8 × 10⁻¹⁰ M) was mixed with a constant concentration of antibody derived from the preliminary ELISA calibration. The incubation was performed in 5 % BSA, 0.05 % Tween-20 in PBS using polypropylene test tubes to minimize antibody loss by adsorption on the microreaction tube walls. After 2 hrs, 100 µl of each mixture was transferred and incubated for 30 min into the wells of a microtiter plate previously coated with biotinylated-(G)₅-Aβ(12-40) (1 µM) and blocked. The concentration of free antibody was then measured by indirect ELISA as described above. The K_{d} values were obtained by plotting the experimental data using the Sips coordinates. The following K_{d} values were determined for the Aβ-antibody complexes of Aβ(12-40) peptide:
   a) serum IVIG; purified: 8 x 10⁻⁹ M
   b) Serum-Ab, Ü-30-A: 14 x 10⁻⁹ M
   c) Serum-Ab, Ü-30-B: 18 x 10⁻⁹ M#

Since the range of binding/dissociation constants for the formation of Aβ-fibrils/aggregates has been estimated in the literature to be in the range of 10⁻⁶ M (determined), the binding of antibodies is determined to be specific. For IgG antibodies, typical K_{d} values in the range of 10⁻⁸ to 10⁻⁹ M have been determined for a large variety of oligo- and polypeptide antigens and epitopes.

## Claims

1. Polypeptide comprising an amino acid sequence of an Aβ peptide, wherein the Aβ peptide has at least the sequence according to SEQ ID NO: 2 and at most the sequence according to SEQ ID NO: 4.

2. The polypeptide of claim 1 further comprising moieties such as tags or markers, in particular biotin, streptavidin, GST, HIS, STREP-tag, Myc, HA, poly-L-lysine, poly-L-lysine-L-alanine copolymers, poly-Aib (alpha-aminoisobutyric acid), poly-β-alanine, poly-L-alanine, poly-D-lysine, poly-D-lysine-D-alanine copolymers, poly-D-alanine, or combinations of poly-L- and -D-amino acids.

3. The polypeptide according to claims 1 or 2, wherein the region of the polypeptide having the sequence according to SEQ ID NO: 2 is not in β-sheet conformation, in particular wherein this region is in random coil or α-helix conformation.

4. The polypeptide according to any one of claims 1 to 3, wherein the region of the polypeptide having the sequence according to SEQ ID NO: 2 is flanked by amino acid sequences, which prevent or reduce β-sheet formation of the polypeptide region having the sequence according to SEQ ID NO: 2.

5. The polypeptide according to claim 4, wherein said flanking amino acid sequences are located in close proximity to the N- and/or C-terminal ends of the polypeptide having the sequence according to SEQ ID NO: 2.

6. The polypeptide according to claim 4 or 5, wherein said flanking amino acid sequences are composed of oligomeric peptides

7. The polypeptide according to any one of claims 4 to 6, wherein the flanking amino acid sequences comprise L-alanine, D-alanine, Aib (alpha-aminoisobutyric acid), β-alanine, D-valine, L-glycine, D-glycine and/or related hydrophobic amino acids,

8. The polypeptide of claim 6 or 7, wherein said flanking amino acid sequences are oligomeric peptides selected from -(L-alanine)ₙ-, -(D-alanine)ₙ-, -(Aib)ₙ-, -(β-alanine)ₙ-, - (D-valine)ₙ-, -(L-glycine)ₙ-, -(D-glycine)ₙ- wherein n ranges from about 2 to about 6.

9. Use of the polypeptide according to anyone of the preceding claims and/or a polypeptide comprising an amino acid sequence of an Aβ peptide, wherein the Aβ peptide has at least the sequence according to SEQ ID NO: 3 and at most the sequence according to SEQ ID NO: 5, for diagnostic assays.

10. A method for diagnosing a neurodementing disease, the method comprising the following steps:
a) incubating a polypeptide according to any one of claims 1 to 8 immobilized on a carrier with a sample derived from a subject,
b) separating said sample from the carrier, and
c) detecting polypeptides bound to said immobilized polypeptide of step a).

11. A method for diagnosing a neurodementing disease, the method comprising the following steps:
a) incubating a polypeptide according to any one of claims 1 to 8 with a sample derived from a subject, and subsequently
b) incubating the sample with a carrier having a binding affinity for said polypeptide according to any one of claims 1 to 8,
c) separating said sample from the carrier, and
d) detecting polypeptides bound to said polypeptide according to any one of claims 1 to 8, said polypeptide being bound to the carrier.

12. A method for diagnosing a neurodementing disease, the method comprising the following steps:
a) incubating a polypeptide immobilized on a carrier with a sample derived from a subject, wherein said polypeptide comprises an amino acid sequence of an Aβ peptide, wherein the Aβ peptide has at least the sequence according to SEQ ID NO: 3 and at most the sequence according to SEQ ID NO: 5,
b) separating said sample from the carrier, and
c) detecting polypeptides bound to said immobilized polypeptide of step a).

13. A method for diagnosing a neurodementing disease, the method comprising the following steps:
a) incubating a polypeptide with a sample derived from a subject, wherein the polypeptide comprises an amino acid sequence of an Aβ peptide, wherein the Aβ peptide has at least the sequence according to SEQ ID NO: 3 and at most the sequence according to SEQ ID NO: 5, and subsequently
b) incubating said sample with a carrier having a binding affinity for said polypeptide comprising an amino acid sequence of an Aβ peptide, wherein the Aβ peptide has at least the sequence according to SEQ ID NO: 3 and at most the sequence according to SEQ ID NO: 5,
c) separating said sample from the carrier, and
d) detecting polypeptides bound to said immobilized polypeptide, which comprises an amino acid sequence of an Aβ peptide, wherein the Aβ peptide has at least the sequence according to SEQ ID NO: 3 and at most the sequence according to SEQ ID NO: 5, said polypeptide being bound to the carrier.

14. The method according to any one of claims 10 to 13, wherein the detection of polypeptides in step c) and d), respectively, is performed by means of ELISA, ELISPOT, Western-Blot, Dot Blot, Protein-Chip, surface plasmon resonance assays, immunoprecipitation or co-immunoprecipitation, or affinity chromatography, in particular immunoaffinity chromatography.

15. A method for diagnosing a neurodementing disease, the method comprising the following steps:
a) incubating a polypeptide according to any one of claims 1 to 8 immobilized on a carrier with a cell containing sample derived from a subject,
b) separating said sample from the carrier, and
c) detecting cells bound to said polypeptide of step a).

16. A method for diagnosing a neurodementing disease, the method comprising the following steps:
a) incubating a polypeptide according to any one of claims 1 to 8 with a cell containing sample derived from a subject, and subsequently
b) incubating the sample with a carrier having a binding affinity for said polypeptide according to any one of claims 1 to 8,
c) separating said sample from the carrier, and
d) detecting cells bound to said polypeptide according to any one of claims 1 to 8, said polypeptide being bound to the carrier.

17. A method for diagnosing a neurodementing disease, the method comprising the following steps:
a) incubating a polypeptide immobilized on a carrier with a cell containing sample derived from a subject, wherein said polypeptide comprises an amino acid sequence of an Aβ peptide, wherein the Aβ peptide has at least the sequence according to SEQ ID NO: 3 and at most the sequence according to SEQ ID NO: 5,
b) separating said sample from the carrier, and
c) detecting cells bound to said immobilized polypeptide of step a).

18. A method for diagnosing a neurodementing disease, the method comprising the following steps:
a) incubating a polypeptide with a cell containing sample derived from a subject, wherein said polypeptide comprises an amino acid sequence of an Aβ peptide, wherein the Aβ peptide has at least the sequence according to SEQ ID NO: 3 and at most the sequence according to SEQ ID NO: 5, and subsequently
b) incubating said sample with a carrier having a binding affinity for said polypeptide comprising an amino acid sequence of an Aβ peptide, wherein the Aβ peptide has at least the sequence according to SEQ ID NO: 3 and at most the sequence according to SEQ ID NO: 5,
c) separating said sample from the carrier, and
d) detecting cells bound to said polypeptide, which comprises an amino acid sequence of an Aβ peptide, wherein the Aβ peptide has at least the sequence according to SEQ ID NO: 3 and at most the sequence according to SEQ ID NO: 5, said polypeptide being bound to the carrier.

19. The method according to any one of claims 15 to 18, wherein the method is carried out as affinity chromatography, in particular immunoaffinity chromatography.

20. The methods according to any one of claims 12, 13, 17 or 18, wherein said polypeptide comprising the Aβ peptide additionally comprises moieties such as tags or markers, in particular biotin, streptavidin, GST, HIS, STREP-tag, Myc, HA, poly-L-lysine, poly-L-lysine-L-alanine copolymers, poly-Aib (alpha-aminoisobutyric acid), poly-β-alanine, poly-L-alanine, poly-D-lysine, poly-D-lysine-D-alanine copolymers, poly-D-alanine, or combinations ofpoly-L- and -D-amino acids.

21. The method according to any one of claims 10, 11, 15 or 16, wherein the method is performed simultaneously, prior or after to a method according to any one of claims 12, 13, 17, 18 or 20.

22. The method according to any one of claims 10, 11, 15, 16 or 21, wherein a solvent is present in the incubation step(s), which prevents or reduces β-sheet formation of the polypeptide region having the sequence according to SEQ ID NO: 2, in particular wherein TFE, hexafluoro-isoporpanol or a similar solvent is present in the incubation step.

23. The method according to 22, wherein the concentration of TFE, hexafluoro-isopropanol or a similar solvent ranges from about 1% to about 5%, in particular from about 1% to about 2%.

24. The method according to any one of claims 10 to 23, wherein at least one washing step is performed before the detecting step.

25. The method according to any one of claims 10 to 14, wherein the polypeptides to be detected are antibodies, in particular an Aβ(21-37) autoantibody or an Aβ(4-10) autoantibody.

26. The method according to any one of claims 10 to 25, wherein said neurodementing disease is selected from Alzheimer's disease, Down's syndrome, Dementia with Lewy bodies, fronto-temporal dementia, cerebral amyloid angiopathy, and/or amyloidoses.

27. A carrier comprising the polypeptide according to any one of claims 1 to 8.

28. The carrier of claim 27, wherein the carrier comprises a second polypeptide comprising an amino acid sequence of an Aβ peptide, wherein the Aβ peptide of the second polypeptide has at least the sequence according to SEQ ID NO: 3 and at most the sequence according to SEQ ID NO: 5.

29. The carrier according to claim 27 or 28 or as used in the methods of claims 10 to 26, wherein the carrier is selected from the group consisting of beads, in particular magnetic beads or sepharose beads, membranes, in particular polyvinylidene fluoride or nitrocellulose membranes, glass, sepharose matrices, gold surfaces, synthetic surfaces, in particular microtiter plates.

30. A kit for the diagnosis of a neurodementing disease, wherein the kit comprises the polypeptide according to any one of claims 1 to 8.

31. The kit according to claim 30, wherein the kit comprises a second polypeptide comprising an amino acid sequence of an Aβ peptide, wherein the Aβ peptide of the second polypeptide has at least the sequence according to SEQ ID NO: 3 and at most the sequence according to SEQ ID NO: 5.

32. The kit according to claims 30 or 31, wherein the kit comprises a carrier, in particular a carrier according to any one of claims 27 to 29.

33. The method according to any one of claims 10 to 26, wherein the sample or the cell containing sample, respectively, is derived from blood, plasma, urine or cerebrospinal fluid (CSF) of a subject.

34. The method according to claim 33, wherein said subject is of human, rodent, bovine, porcine, canine or avian origin, in particular wherein said subject is a human, mouse, rat, rabbit, cow, pig, dog or chicken.

35. The method according to claim 33 or 34, wherein the cell containing sample is obtained from human blood and the cells are of the B-cell lineage.

36. A method for diagnosing a neurodementing disease, the method comprising the following steps:
i) performing a first method according to anyone of claims 10 to 26,
ii) performing a second method according to anyone of claims 10 to 26 proviso that the polypeptide to be incubated in step a) of said second method comprises the full length amino acid sequence of Aβ peptide, and
iii) comparing the result obtained from step i) with the result of step ii).
